# EUROPÄISCHE PATENTSCHRIFT

(11) **EP 2 854 789 B1**
(45) Veröffentlichungstag und Bekanntmachung des Hinweises auf die Patenterteilung: **26.06.2019**
(21) Anmeldenummer: 13725157.5
(22) Anmeldetag: 27.05.2013
(51) Int. Cl.: A61K 31/138, A61P 35/00

(54) **GENOTYP- BZW. PHÄNOTYP-BASIERTE ARZEIMITTELFORMULIERUNGEN**
GENOTYPE AND PHENOTYPE-BASED MEDICINAL FORMULATIONS
FORMULATIONS DE MÉDICAMENT BASÉES SUR UN GÉNOTYPE OU UN PHÉNOTYPE

(30) Priorität: 01.06.2012 EP 12170401
(43) Veröffentlichungstag der Anmeldung: 08.04.2015
(73) Patentinhaber: Bayer Aktiengesellschaft, 51373 Leverkusen (DE)
(72) Erfinder: WILLMANN, Stefan, 40593 Düsseldorf (DE); EIßING, Thomas, 40764 Langenfeld (DE); DICKSCHEN, Kristin, 40545 Düsseldorf (DE)
(74) Vertreter: BIP Patents
(86) Internationale Anmeldenummer: PCT/EP2013/060824
(87) Internationale Veröffentlichungsnummer: WO 2013/178565

(56) Entgegenhaltungen:
- EP-A2- 0 207 451
- BLONDEAU J M ET AL: "In vitro killing of Escherichia coli, Staphylococcus pseudintermedius and Pseudomonas aeruginosa by enrofloxacin in combination with its active metabolite ciprofloxacin using clinically relevant drug concentrations in the dog and cat.", VETERINARY MICROBIOLOGY 23 MAR 2012 LNKD- PUBMED:21925810, Bd. 155, Nr. 2-4, 23. März 2012 (2012-03-23), Seiten 284-290, XP008157009, ISSN: 1873-2542
- VALLÉE E ET AL: "Individual and combined activities of clarithromycin and its 14-hydroxy metabolite in a murine model of Haemophilus influenzae infection", JOURNAL OF ANTIMICROBIAL CHEMOTHERAPY, OXFORD UNIVERSITY PRESS, GB, Bd. 27, Nr. Suppl. A, 1. Februar 1991 (1991-02-01), Seiten 31-41, XP008157004, ISSN: 0305-7453, DOI: 10.1093/JAC/27.SUPPL_A.31
- ROSS A CATHARINE ET AL: "The components of VARA, a nutrient-metabolite combination of vitamin A and retinoic acid, act efficiently together and separately to increase retinyl esters in the lungs of neonatal rats.", THE JOURNAL OF NUTRITION NOV 2006 LNKD- PUBMED:17056804, Bd. 136, Nr. 11, November 2006 (2006-11), Seiten 2803-2807, XP055040336, ISSN: 0022-3166
- MARTIN S J ET AL: "In vitro activity of clarithromycin alone and in combination with ciprofloxacin or levofloxacin against Legionella spp.: enhanced effect by the addition of the metabolite 14-hydroxy clarithromycin", DIAGNOSTIC MICROBIOLOGY AND INFECTIOUS DISEASES, ELSEVIER SCIENCE PUBLISHING CO., AMSTERDAM, NL, Bd. 29, Nr. 3, 1. November 1997 (1997-11-01), Seiten 167-171, XP002536389, ISSN: 0732-8893, DOI: 10.1016/S0732-8893(97)81806-8
- AHMAD A ET AL: "Endoxifen, a new cornerstone of breast cancer therapy: demonstration of safety, tolerability, and systemic bioavailability in healthy human subjects.", CLINICAL PHARMACOLOGY AND THERAPEUTICS DEC 2010 LNKD- PUBMED:20981001, Bd. 88, Nr. 6, Dezember 2010 (2010-12), Seiten 814-817, XP008157006, ISSN: 1532-6535
- DATABASE MEDLINE [Online] US NATIONAL LIBRARY OF MEDICINE (NLM), BETHESDA, MD, US; Mai 2011 (2011-05), MADLENSKY L ET AL: "Tamoxifen metabolite concentrations, CYP2D6 genotype, and breast cancer outcomes.", XP002685041, Database accession no. NLM21430657 & MADLENSKY L ET AL: "Tamoxifen metabolite concentrations, CYP2D6 genotype, and breast cancer outcomes.", CLINICAL PHARMACOLOGY AND THERAPEUTICS MAY 2011 LNKD- PUBMED:21430657, Bd. 89, Nr. 5, Mai 2011 (2011-05), Seiten 718-725, ISSN: 1532-6535
- HERTZ DANIEL L ET AL: "Tamoxifen and CYP2D6: a contradiction of data", THE ONCOLOGIST, ALPHAMED PRESS, INC, UNITED STATES, Bd. 17, Nr. 5, 1. Mai 2012 (2012-05-01), Seiten 620-630, XP008156998, ISSN: 1549-490X, DOI: 10.1634/THEONCOLOGIST.2011-0418 [gefunden am 2012-04-24]
- ALAN H B WU ET AL: "Estimation of tamoxifen metabolite concentrations in the blood of breast cancer patients through CYP2D6 genotype activity score", BREAST CANCER RESEARCH AND TREATMENT, KLUWER ACADEMIC PUBLISHERS, BO, Bd. 133, Nr. 2, 1. Februar 2012 (2012-02-01), Seiten 677-683, XP035062641, ISSN: 1573-7217, DOI: 10.1007/S10549-012-1963-2
- Anonymous: "Cancer: Tamoxifen's Power Comes From Endoxifen -- ScienceDaily", Science Daily, 24 December 2008 (2008-12-24), XP0055407677, Retrieved from the Internet: URL:https://www.sciencedaily.com/releases/ 2008/12/081211141838.htm [retrieved on 2017-09-19]
- T E Mürdter ET AL: "Activity Levels of Tamoxifen Metabolites at the Estrogen Receptor and the Impact of Genetic Polymorphisms of Phase I and II Enzymes on Their Concentration Levels in Plasma", Clinical Pharmacology and Therapeutics, vol. 89, no. 5, 30 March 2011 (2011-03-30) , pages 708-717, XP055436740, US ISSN: 0009-9236, DOI: 10.1038/clpt.2011.27

## Beschreibung

Die Erfindung betrifft eine Kombination aus zweien oder mehreren pharmazeutisch aktiven Substanzen, von denen mindestens eine ein Stoffwechselprodukt ("Metabolit") der anderen ("Muttersubstanz") ist, insbesondere deren Dosierungen derart gewählt werden, dass eine geno- bzw. phänotypisch (Definition Genotyp http://de.wikipedia.org/wiki/Genotyp, Definition Phänotyp: http://de.wikipedia.org/wiki/Ph%C3%A4notyp) bedingte Variabilität der Umsetzung der Muttersubstanz zu dem/der Metaboliten in bestimmten Individuen kompensiert wird.

Die Erfindung betrifft genauer eine Kombination des Brustkrebsmedikaments Tamoxifen und dessen aktiven Metaboliten Endoxifen wie in den beiliegenden Ansprüchen definiert.

In der Pharmakotherapie gibt es zahlreiche Beispiele für Arzneimittel, deren pharmakologische Wirkung sich aus dem Zusammenspiel von der verabreichten Muttersubstanz mit im Körper des Patienten entstehenden Metaboliten ergibt. Die Bildung solcher sogenannten aktiven Metaboliten erfolgt in der Regel über enzymatisch katalysierte Prozesse, welche z. B. in der Leber, der Niere, dem Darm oder jedem anderen Organ des Körpers stattfinden können. Die Aktivität dieser enzymatischen Prozesse kann bei verschiedenen Individuen stark unterschiedlich ausgeprägt sein. Die Gründe für individuell unterschiedlich ausgeprägte Enzymaktivitäten sind dabei vielfältiger Natur. Zum einen gibt es individuelle Schwankungen in der Menge der exprimierten Enzymvariante, die z. B. durch Enzyminhibitoren oder -induktoren oder aber durch genetische Ursachen hervorgerufen werden können. Zum anderen gibt es individuelle Schwankungen in der Aktivität der exprimierten Enzymvariante, die z.B. durch Enzyminhibitoren oder -induktoren oder aber durch genetische Ursachen auftreten können. Viele pharmazeutische Wirkstoffe sind bekannte Cytochrom P450 Enzyminhibitoren, wie beispielsweise:
2-(4-chlorphenoxy-)ethanol, Acarbose, Acebutolol, Acenocoumarol, Acetazolamid, Adefovir, Ademethionin, Ajmalin, Albendazol, Alitretinoin, Allopurinol, Alosetron, Ambroxol, Amphetamin, Amilorid, Aminoglutethimid, Aminophenazon, Amiodaron, Amitriptylin, Amlodipin, Amodiaquin, Amprenavir, Anastrozol, Androstandolon, Aprepitant, Aripiprazol, Arsentrioxid, Artemisinin, Artesunat, Astemizol, Atazanavir, Atomoxetin, Atorvastatin, Atovaquon, Atropin, Azapropazon, Azelastin, Azithromycin, Barnidipin, Benazepril, Benidipin, Benzbromaron, Benzethonium, Benzocain, Bergapten, Betamethason, Betaxolol, Bezafibrat, Bicalutamid, Bifonazol, Biperiden, Bortezomib, Bromazepam, Bromocriptin, Brompheniramin, Budipin, Buprenorphin, Buproprion, Calcitriol, Candesartan, Capecitabin, Carbamazepin, Carbinoxamin, Carteolol, Caspofungin, Celecoxib, Cerivastatin, Chinidin, Chinin, Chloramphenicol, Chlormadinon, Chloroquin, Chlorphenamin, Chlorpromazin, Chlorzoxazon, Ciclosporin, Cimetidin, Ciprofibrat, Ciprofloxacin, Cisaprid, Cisplatin, Citalopram, Clarithromycin, Clemastin, Clevidipin, Clindamycin, Clobetasol, Clofamizin, Clofenotan, Clofibrat, Clomethiazol, Clomifen, Clomipramin, Clonazepam, Clopidogrel, Clotiazepam, Clotrimazol, Clozapin, Cocain, Codein, Coffein, Colchicin, Colecalciferol, Cyclizin, Cylcophosphamid, Cyproteron, Dacarbazin, Dactinomycin, Dalfopristin, Danazol, Dantrolen, Daunorubicin, Deferoxamin, Delarvirdin, Desipramin, Desloratadin, Desvenlafaxin, Dexamethason, Dexamfetamin, Dexfenfluramin, Dexibuprofen, Dextrometorphan, Dextropropoxyphen, Diazepam, Diclofenac, Dicoumarol, Dihydralazin, Dihydroergotamin, Diiodohydroxypropan, Diltiazem, Dimethylsulfoxid, Dimetotiazin, Diosmectit, Diosmin, Diphenhydramin, Disulfiram, Docetaxel, Dolasetron, Dopamin, Doxepin, Doxorubicin, Doxycyclin, Ebastin, Econazol, Efavirenz, Emetin, Enoxacin, Enoxolon, Enprostil, Entacapon, Epinastin, Epinephrin, Eplerenon, Eprosartan, Ergometrin, Ergotamin, Erythromycin, Escitalopram, Estriol, Etanautin, Ethanol, Ethinylestradiol, Ethotoin, Etodolac, Etomidat, Etoposid, Etoricoxib, Etretinat, Exemestan, Ezetimib, Felbamat, Felodipin, Fenfluramin, Fenofibrat, Fentanyl, Fexofenadin, Flecainid, Fluconazol, Flumequin, Fluoruracil, Fluoxetin, Fluphenazin, Flurazepam, Flurbiprofen, Flurithromycin, Flutamid, Fluvastatin, Fluvoxamin, Fomepizol, Formestan, Fosamprenavir, Fosphenytoin, Gefitinib, Gemfibrozil, Glibenclamid, Gliclazid, Glucose, Glutethimid, Granisetron, G-Strophantin, Halofantrin, Haloperidol, Histamin, Hydralazin, Hydrocortison, Hydroxycarbamid, Hydroxychloroquin, Hydroxyzin, Ibuprofen, Idarubicin, Ifosfamid, Imatinib, Imipramin, Indinavir, Indometacin, Insulin, Ipriflavon, Irbesartan, Irinotecan, Isoconazol, Isofluran, Isoniazid, Isoprenalin, Isopropanol, Isosorbiddinitrat, Isradipin, Itraconazol, Josamycin, Ketoconazol, Ketoprofen, Labetalol, Lafutidin,, Lansoprazol, Leflunomid, Lentinan, Lercarnidipin, Letrozol, Levofloxacin, Levomepromazin, Levonorgestrel, Lidocain, Lomefloxacin, Lomustin, Loperamid, Lopinavir, Loratadin, Lornoxicam, Losartan, Lovastatin, Manidipin, Masoprocol, Meclozin, Medazepam, Medroxyprogesteron, Medryson, Mefenaminsäure, Mefloquin, Meglutol, Melatonin, Meloxicam, Melperon, Memantin, Menadion, Mephenytoin, Mequitazin, Mesuximid, Metamfetamin, Metformin, Methadon, Methazolamid, Methoxsalen, Methylphenidat, Methylphenobarbital, Methylprednisolon, Metoclopramid, Metoprolol, Metronidazol, Metyrapon, Mexiletin, Mianserin, Mibefradil, Miconazol, Midazolam, Midecamycin, Midodrin, Mifepriston, Minoxidil, Miocamycin, Mirtazapin, Mitoxantron, Mizolastin, Moclobemid, Modafinil, Mometason, Montelukast, Moracizin, Nefazodon, Nelfinavir, Neostigmin, Nevirapin, Nicardipin, Niclosamid, Nicotinamid, Nifedipin, Nikotin, Nikotinsäure, Nilutamid, Nilvadipin, Nimesulid, Nisoldipin, Nitrendipin, Nitroprussid, Norepinephrin, Norfloxacin, Nortriptylin, Noscapin, Octopamin, Ofloxacin, Olanzapin, Oleandomycin, Omeprazol, Ondansetron, Orphenadrin, Oxamniquin, Oxatomid, Oxcarbazepin, Oxprenolol, Oxybutynin, Oxycodon, Paclitaxel, Pancreozymin (Cholecystokinin), Pantoprazol, Paracetamol, Parecoxib, Pargylin, Paroxetin, Pazopanib, Pefloxacin, Pentoxyverin, Perazin, Pergolid, Perhexilin, Perphenazin, Phenazon, Phenelzin, Phenobarbital, Phensuximid, Phentermin, Phenylbutazon, Phenylpropanolamin, Phenytoin, Physostigmin, Pilocarpin, Pimozid, Pindolol, Pioglitazon, Piroxicam, Pranlukast, Prasteron, Pravastatin, Praziquantel, Prednisolon, Prednison, Primaquin, Pristinamycin, Probenecid, Progesteron, Proguanil, Promethazin, Propafenon, Propanol, Propiverin, Propofol, Propranolol, Pyrimethamin, Quassia, Quecksilber, Quetiapin, Quinidin, Quinin, Quinopristin, Rabeprazol, Raloxifen, Ranitidin, Reboxetin, Retinol, Rifampicin, Risperidonl, Ritonavir, Rivastigmin, Rofecoxib, Rokitamycin, Ropinirol, Rosiglitazon, Rosuvastatin, Roxithromycin, Rutosid, Salbutamol, Salicylamid, Salmeterol, Saquinavir, Selegilin, Seratrodast, Sertaconazol, Sertralin, Sildenafil, Silymarin, Simvastatin, Sirolimus, Somatostatin, Sorbitol, Spartein, Spironolacton, Stickstoffmonoxid, Sulconazol, Sulfadiazin, Sulfadimethoxin, Sulfadimidin, Sulfafurazol, Sulfamethizol, Sulfamethoxazol, Sulfamoxol, Sulfanilamid, Sulfaphenazol, Sulfapyridin, Sulfinpyrazon, Sulindac, Sulpirid, Suprofen, Tacrolimus, Tamoxifen, Tegaserod, Telithromycin, Telmisartan, Temafloxacin, Teniposid, Tenofovir, Terbinafin, Terconazol, Terfenadin, Teriparatid, Testosteron, Tetracyclin, Theophyllin, Thiamazol, Thiopental, Thioridazin, Thiosulfat, Thiotepa, Tiabendazol, Tibolon, Ticlopidin, Timolol, Tinidazol, Tioconazol, Tiopronin, Tiotixen, Tocainid, Tocopherol, Tofisopam, Tolbutamid, Tolcapon, Topiramat, Topotecan, Torasemid, Tramadol, Tranylcypromin, Trastuzumab, Treosulfan, Tretinoin, Triamteren, Triazolam, Trichloroethylen, Triclosan, Trimethoprim, Tripelennamin, Triprolidin, Troglitazon, Troleandomycin, Tropisetron, Trospium, Ursodeoxycholinsäure, Valdecoxib, Valproinsäure, Valsartan, Venlafaxin, Verapamil, Vinblastin, Vincristin, Vinorelbin, Virginiamycin, Voriconazol, Vorozol, Warfarin, Yohimbin, , Zafirlukast, Ziprasidon, Zolpidem, Zonisamid.

Besonders hervorzuheben sind hierbei: Fluvoxamin, Ciprofloxacin, Gemfibrozil, Fluconazol, Bupropion, Cinacalcet, Fluoxetin, Paroxetin, Chinidin, Indinavir, Nelfinavir, Ritonavir, Clarithromycin, Itraconazol, Ketoconazol, Nefazodon, Saquinavir, Telithromycin, Trimethoprim, Amiodaron, Duloxetin, Sertralin, Terbinafin, Aprepitant, Erythromycin, , **Verapamil**, **Diltiazem**, **Cimetidin**, **Amiodaron** [http://medicine.iupui.edu/clinpharm/ddis/table.aspx Stand 09.05.2012].

Bekannte Inhibitoren von Phase 2 Enzymen sind unter anderem:
Acarbose, Acetylcholin, Acetylsalicylsäure, Amitriptylin, Apomorphin, Artemisinin, Ascorbinsäure, Bendroflumethiazid, Bergapten, Bromocriptin, Carbachol, Carbamazepin, Carmustin, Celecoxib, Chenodesoxycholsäure, Chinin, Chlorhexidin, Chloroquin, Cimetidin, Clomipramin, Clonidin, Cocain, Cortison, Dactinomycin, Desipramin, Diazepam, Dicoumarol, Dicycloverin, Diosmin, Disulfiram Doxepin, Enoxolon, Entacapon, Estradiol, Etacrynsäure, Fluconazol, Fluphenazin, Folsäure, Haloperidol, Hematin, Hydrocortison, Hymecromon, Ibuprofen, Imipramin, Indometacin, Iproniazid, Ketoprofen, Lidocain, Lopinavir, Medroxyprogesteron, Melatonin, Mepacrin, Mercaptamin, Mersalyl, Mesalazin, Methyldopa, Moclobemid, Naproxen, Natriumcitrat, Natriumsalicylat, Nifluminsäure, Nikotin, Olsalazin, Oxedrin, Paclitaxel, Pargylin, Phenylbutazon, Physostigmin, Pipamperon, Polihexanid, Primaquin, Probenecid, Progesteron, Propylthiouracil, Pyridoxal, Pyridoxin, Pyrimethamin, Ranitidin, Ritonavir, Salicylamid, Salicylsäure, Saquinavir, Silymarin, Sulfobromophthalein, Sulindac, Tacrin, Tamoxifen, Tetracyclin, Thiomersal, Tolcapone, Triclosan, Tubocurarin, Vecuronium, Warfarin, Wasserstoffperoxid.

Bekannte Cytochrom P450 Enzyminduktoren sind beispielsweise:
2-(4-chlorphenoxy-)-Ethanol, Acarbose, Acetylsalicylsäure, Acriflaviniumchlorid, Albendazol, Aldosteron, Alum, Aminogluthetimid, Aminosalicylsäure, Amobarbital, Angiotensinamid, Aprepitant, Aprobarbital, Aripiprazol, Artemisinin, Ascorbinsäure, Azatidin, Beclometason, Benoxaprofen, Betakaroten, Betamethason, Bexaroten, Bezafibrat, Biotin, Bosentan, Bucladesin, Buserelin, Captopril, Carbamazepin, Carbamid, Carboplatin, Chinidin, Chinin, Chlordiazepoxid, Chlorothiazid, Chlorpromazin, Ciclosporin, Ciprofibrat, Ciprofloxacin, Cisplatin, Clacitriol, Clarithromycin, Clofenotan, Clofibrat, Clomifen, Clonazepam, Clonidin, Clotrimazol, Clozapin, Colchicin, Colestyramin, Corticotropin, Cyclobarbital, Cyclophosphamid, Dapson, Daunorubicin, Dexamethason, Dextropropoxyphen, Diazepam, Dibutylphtalat, Diclofenamid, Dicloxacillin, Dicycloverin, Diethylether, Diethylstilberol, Diiodohydroxypropan, Dinoproston, Diosmectit, Diosmin, Docetaxel, Doxorubicin, Doxylamin, Efavirenz, Eletriptan, Enoxacin, Ergocalciferol, Erythromycin, Estriol, Ethanol, Ethinylestradiol, Etoposid, Febendazol, Felbamat, Fluconazol, Flucoxacillin, Flufenaminsäure, Flurescein, Fluvastatin, Gemfibrozil, Glukose, Gluthation, Glycerol, Glycyrhizzinsäure, Granisetron, Griseofulvin, Guanethidin, Haloperidol, Histamin, Hydrocortison, Hydroxycarbamid, Ifosfamid, Insulin, Ipriflavon, Isofluran, Isoniazid, Isoprenalin, Isopropanol, Itraconazol, Ketoconazol, Kokain, Lansoprazol, Lindan, Loratadin, Lovastatin, Lynestrenol, Mebendazol, Mecamylamin, Medroxyprogesteron, Metamizol, Methadon, Metharbital, Methohexital, Methylprednisolon, Methyltestosteron, Metoclopramid, Metyrapon, Mifepriston, Mirtazapin, Mitobronitol, Mitomycin, Mitotan, Moclobemid, Modafinil, Natriumchlorid, Natriumsalicylat, Nelfinavir, Nevirapin, Nicardipin, Nicotinamid, Nifedipin, Nikotin, Nitrazepam, Norethisteron, Omeprazol, Ondansetron, Oxcarbazepin, Oxiconazol, Oxolamin, Oxomemazin, Paclitaxel, Pantoprazol, Paracetamol, Permethrin, Pethidin, Phenobarbital, Phenoxymethylpenicillin, Phentermin, Phenylbutazon, Phenylephrin, Phenytoin, Pindolol, Pioglitazon, Pipamperon, Pleconaril, Prednisolon, Prednison, Primaquin, Primidon, Pristinamycin, Probenecid, Progesteron, Propylthiouracil, Pyridostigmin, Pyridoxin, Quecksilber, Quinine, Rabeprazol, Reboxetin, Reserpin, Retinol, Rifabutin, Rifampicin, Rifapentin, Rifaximin, Ritonavir, Rofecoxib, Salicylsäure, Secobarbital, Seratrodast, Silymarin, Spironolacton, Streptozocin, Sulfadimidin, Sulfinpyrazon, Tamoxifen, Temozolomid, Terbinafin, Terfenadin, Testosteron, Tetrabenazin, Tetramethrin, Thalidomid, Thiamin, Thiram, Tiabendazol, Tienilinsäure, Tocopherol, Topiramat, Topotecan, Tretinoin, Triamcinolonacetonid, Triamcinolon, Troglitazon, Troglitazon, Tryptophan, Ursodeoxycholinsäure, Valproinsäure, Verapamil, Vinblastin, Virginiamycin, Voglibose.

Besonders hervorzuheben sind hierbei: Modafinil, Nafcillin, Omeprazol, Phenobarbital, Phenytoin, Rifampin, Secobarbital, Carbamazepin, Norethindron, Prednison, Rifampicin, Dexamethason, Isoniazid, Efavirenz, Nevirapin, Barbiturate, Glucocorticoide, Oxcarbazepin, Pioglitazon, Rifabutin, Troglitazon [http://medicine.iupui.edu/clinpharm/ddis/table.aspx Stand 09.05.2012].

Zu den bekannten Induktoren von Phase 2 Enzymen zählen unter anderem:
Acetylcholin, Acetylsalicylsäure, Adenosin, Amfetamin, Aminophyllin, Androstandolon, Angiotensinamid, Argatroban, Ascorbinsäure, Benfluorex, Betacaroten, Betamethason, Bucladesin, Calcitriol, Carbamazepin, Chlorambucil, Chlorphenamin, Cisaprid, Cisplatin, Clofibrat, Clozapin, Cocain, Corticotropin, Desipramin, Dexamethason, Dexamfetamin, Diazepam, Diclofenac, Diethylcarbamazin, Diethylether, Dinoproston, Disulfiram, Doxorubicin, Entacapon, Epinephrin, Esketamin, Estradiol, Estriol, Ethanol, Flunarizin, Fluoxetin, Gabapentin, Glyceryltrinitrat, Glycin, G-Strophantin, Hydralazin, Hydrocortison, Hymecromon, Ibuprofen, Imipramin, Indometacin, Insulin, Isoprenalin, Ketamin, Lamotrigin, Levetiracetam, Levodopa, Lindan, Melatonin, Melphalan, Mequinol, Metamizol, Methionin, Methotrexat, Metoclopramid, Nabumeton, Nandrolon, Norepinephrin, Olanzapin, Paracetamol, Pargylin, Phenobarbital, Phenytoin, Pipamperon, Progesteron, Promegeston, Propylthiouracil, Retinol, Rofecoxib, Spironolacton, Stickstoffmonoxid, Sulindac, Sultiam, Tamoxifen, Testosteron, Theophyllin, Tiadenol, Tibolon, Tioguanin, Triamcinolon, Trimethoprim, Troglitazon, Valproinsäure, Verapamil, Warfarin, Wasserstoffperoxid.

[http://bioinformatics.charite.de/supercyp Stand 24.04.2012]. Neben pharmazeutischen Wirkstoffen können auch Nahrungsbestandteile inhibitorische und/oder induktorische Effekte auf Enzyme, Transporter, Rezeptoren oder andere Proteine haben.

Bekannte Beispiele hierfür sind unter anderem: Brokkoli, Grillfleisch, Johanniskraut, Tabakrauch, Käse, Rotwein, Grapefruitsaft, Folsäure, Vitamin K, Vitamin E, Vitamin B6 und Johanniskraut [Gröber, U. (2009) "Interaktionen Arzneimittel und Mikronährstoffe für die Kitteltasche" Wissenschaftliche Verlagsgesellschaft mbH Stuttgart; Wentworth, J. M., M. Agostini, et al. (2000). "St John's wort, a herbal antidepressant, activates the steroid X receptor." J Endocrinol 166(3): R11-16., http://medicine.iupui.edu/clinpharm/ddis/table.aspx Stand 09.05.20120]. Ähnlich der induktorischen Wirkung des Grillfleisches auf Cytochrom P450 1A1 (CYP1A1) lässt sich das Enzym auch durch polyzyklische Aromate induzieren, die im Zigarettenrauch vorhanden sind. So ist in der Literatur beschrieben, dass die Aktivität von CYP1A1 in der Lunge, Leber und Darm von Rauchern proportional zu deren Zigarettenkonsum erhöht ist [Czekaj, P., A. Wiaderkiewicz, et al. (2005). "Tobacco smokedependent changes in cytochrome P450 1A1, 1A2, and 2E1 protein expressions in fetuses, newborns, pregnant rats, and human placenta." Arch Toxicol 79(1): 13-24.; Fontana, R. J., K. S. Lown, et al. (1999). "Effects of a chargrilled meat diet on expression of CYP3A, CYP1A, and P-glycoprotein levels in healthy volunteers." Gastroenterology 117(1): 89-98.; Kim, J. H., M. E. Sherman, et al. (2004). "Expression of cytochromes P450 1A1 and 1B1 in human lung from smokers, non-smokers, and ex-smokers." Toxicol Appl Pharmacol 199(3): 210-219.,Pelkonen, O., M. Pasanen, et al. (1986). "The effect of cigarette smoking on 7-ethoxyresorufin O-deethylase and other monooxygenase activities in human liver: analyses with monoclonal antibodies." Br J Clin Pharmacol 22(2): 125-134.; Zevin, S. and N. L. Benowitz (1999). "Drug interactions with tobacco smoking. An update." Clin Pharmacokinet 36(6): 425-438.].

Die pharmakologische Wirkung von der Muttersubstanz und ihrem/ihren Metaboliten kann darüber hinaus auch abhängig sein von der Menge bzw. der Aktivität von exprimierten Proteinvarianten, Rezeptorvarianten oder Transportervarianten, welche ebenfalls von Individuum zu Individuum oder innerhalb eines Individuums aufgrund von Inhibition bzw. Induktion oder genetischer Ursachen stark unterschiedlich sein können.

Beispiele für Transporterinduktoren sind: Dexamethason, Doxorubicin, Flavonoide, Johanniskraut, Phenobarbital, Phenytoin, Rifampicin, Vinblastin.

Beispiele für Transporterinhibitoren sind:
Rifampicin, Cyclosporin A, Gemfibrozil, Lopinavir, Ritonavir, Clarythromycin, Furosemid, Indomethacin, Probenecid, Naproxen, Ibuprofen, Piroxicam, Acetylsalicylsäure, Paracetamol, Phenacetin, Ketoprofen, Enalapril, Bumetanid, Cefperazon, Azathioprin, Methotrexat, Valproat, Flufenamat, Phenylbutazon, Levofloxacin, Dexamethason, Cytarabin, Ampicillin, Amoxicillin, Cyclacillin, Cephalexin, Cefadroxil, Cephradin, Cefdinir, Ceftibuten, Cefixim, Captopril, Amiodaron, Chinidin, Lidoacin, Itraconazol, Ketoconazol, Diltiazem, Felodipin, Nicardipin, Nifedipin, Nitrendipin, Verapamil, Indinavir, Nelfinavir, Saquinavir, Ethinylestradiol, Norgestrel, Progesteron, Testosteron, Tacrolimus, Erythromycin, Mifepriston, Paroxetin, Talinolol, Tamoxifen, Terfenadin, Trifluoperazin, Vincristin.
[Shitara, Y. (2011). "Clinical importance of OATP1B1 and OATP1B3 in drug-drug interactions." Drug Metab Pharmacokinet 26(3): 220-227.; Van Aubel, R. A., R. Masereeuw, et al. (2000). "Molecular pharmacology of renal organic anion transporters." Am J Physiol Renal Physiol 279(2): F216-232.; http://www.pharmazeutischezeitung.de/index.php?id=2381].

Von besonderer Bedeutung für die Pharmakotherapie sind solche Unterschiede in der Proteinaktivität, die eine genetische Ursache haben. Durch Sequenzvariationen (http://de.wikipedia.org/wiki/Polymorphismus) in den Allelen und/oder durch eine unterschiedliche Zahl an vorliegenden Allelen können unterschiedliche Varianten und/oder Mengen eines Proteins exprimiert werden. Beides, die exprimierte Variante und die exprimierte Menge eines Proteins, können einen starken Einfluss auf die Aktivität der Proteinvariante haben.

Ein in der Literatur gut untersuchtes Beispiel für ein polymorphes Protein ist das Cytochrom P450 2D6 (CYP2D6), ein Enzym für welches eine Vielzahl unterschiedlicher Genvarianten bekannt ist, die sich in vier verschiedene Phänotypen klassifizieren lassen. Die hierfür üblichen Bezeichnungen sind: PM = "poor metabolizer", IM = "intermediate metabolizer", EM = "extensive metabolizer" und UM = "ultrarapid metabolizer" [Zanger, U. M., J. Fischer, et al. (2001). "Comprehensive analysis of the genetic factors determining expression and function of hepatic CYP2D6." Pharmacogenetics 11(7): 573-585].

Neben dem CYP2D6 gibt es zahlreiche andere polymorphe Enzyme aus der Klasse der Cytochrom P450 (CYP) Isoenzyme:
CYP1A1, CYP1A2, CYP1B1, CYP2A6, CYP2A13, CYP2B6, CYP2C8, CYP2C9, CYP2C11, CYP2C18, CYP2C19, CYP2D6, CYP2E1, CYP2F1, CYP2J2, CYP2S1, CYP2W1, CYP3A4, CYP3A5, CYP3A7, CYP3A43, CYP4A11, CYP4B1, CYP4F2, CYP4F22, CYP7A1, CYP4B1, CYP7B1, CYP8A1, CYP8B1, CYP11A, CYP11B1, CYP11B2, CYP17A, CYP19A, CYP21A, CYP24A, CYP26A1, CYP26B, CYP27A, CYP27B, CYP46A, CYP51A.

Besonders hervorzuheben sind hierbei: CYP1A2, CYP2B6, CYP2C8, CYP2C9, CYP2C19, CYP2D6, CYP2E1, CYP3A4, CYP3A5, CYP3A7 [http://bioinformatics.charite.de/supercyp Stand 24.04.2012; Tamaki, Y., T. Arai, et al. (2011). "Association between cancer risk and drug-metabolizing enzyme gene (CYP2A6, CYP2A13, CYP4B1, SULT1A1, GSTM1, and GSTT1) polymorphisms in cases of lung cancer in Japan." Drug Metab Pharmacokinet 26(5): 516-522.].

Ebenso existieren zahlreiche polymorphe Phase 2 Enzyme bzw. andere Enzyme im Stoffwechsel, wie beispielsweise:
N-Acetyltranferase 2 (NAT2), Thiopurin-S-Methyltransferase (TPMT), Uridin-5'-diphospho-glucuronosyltransferase (UGT) 1A1, UGT1A3, UGT1A4, UGT1A5, UGT1A6, UGT1A7, UGT1A8, UGT1A9, UGT1A10, UGT2A1, UGT2A2, UGT2A3, UGT2B4, UGT2B7, UGT2B10, UGT2B15, UGT2B17, Sulfonyltransferase (SULT) 1A1, SULT1A2, SULT1A3, SULT1E1, SULT2A1, SULT2B1, SULT4A1, Glutathion-S-Transferase (GST) A1, GSTA2, GSTA3, GSTA4, GSTA5, GSTM1, GSTM2, GSTM3, GSTM4, GSTM5, GSTP1, GSTT1, GSTT2, GSTO1, GSTO2, Catechol-o-Methyltransferase (COMT), Flavin-abhängige Monooxygenase 3 (FMO), Dihydropyrimidin-Dehydrogenase (DPD), Methylentetrahydrofolat-Reduktase (MTHFR).

Besonders hervorzuheben sind hierbei: NAT2, TPMT, UGT1A1, UGT1A4, UGT2B7, UGT2B15, SULT1A1, SULT1A2, SULT2A1, GSTM1, GSTP1, GSTT1, COMT, DPD, MTHFR [Hickman, D. and E. Sim (1991). "N-acetyltransferase polymorphism. Comparison ofphenotype and genotype in humans." Biochem Pharmacol 42(5): 1007-1014.; Yates, C. R., E. Y. Krynetski, et al. (1997). "Molecular diagnosis of thiopurine S-methyltransferase deficiency: genetic basis for azathioprine and mercaptopurine intolerance." Ann Intern Med 126(8): 608-614.; Bernard, O., J. Tojcic, et al. (2006). "Influence of nonsynonymous polymorphisms of UGT1A8 and UGT2B7 metabolizing enzymes on the formation of phenolic and acyl glucuronides of mycophenolic acid." Drug Metab Dispos 34(9): 1539-1545.; Bushey, R. T., G. Chen, et al. (2011). "Characterization of UDP-glucuronosyltransferase 2A1 (UGT2A1) variants and their potential role in tobacco carcinogenesis." Pharmacogenet Genomics 21(2): 55-65.; Carlini, L. E., N. J. Meropol, et al. (2005). "UGT1A7 and UGT1A9 polymorphisms predict response and toxicity in colorectal cancer patients treated with capecitabine/irinotecan." Clin Cancer Res 11(3): 1226-1236.; Chen, G., A. S. Blevins-Primeau, et al. (2007). "Glucuronidation of nicotine and cotinine by UGT2B10: loss of function by the UGT2B10 Codon 67 (Asp>Tyr) polymorphism." Cancer Res 67(19): 9024-9029.; Chen, G., R. W. Dellinger, et al. (2008). "Identification of a prevalent functional missense polymorphism in the UGT2B10 gene and its association with UGT2B10 inactivation against tobacco-specific nitrosamines." Pharmacogenet Genomics 18(3): 181-191.; Chen, Y., S. Chen, et al. (2006). "Genetic variants of human UGT1A3: functional characterization and frequency distribution in a Chinese Han population." Drug Metab Dispos 34(9): 1462-1467.; Dellinger, R. W., J. L. Fang, et al. (2006). "Importance of UDP-glucuronosyltransferase 1A10 (UGT1A10) in the detoxification of polycyclic aromatic hydrocarbons: decreased glucuronidative activity of the UGT1A10139Lys isoform." Drug Metab Dispos 34(6): 943-949.; Guo, Y., C. Hu, et al. (2012). "Effects of UGT1A6, UGT2B7, and CYP2C9 genotypes on plasma concentrations of valproic acid in Chinese children with epilepsy." Drug Metab Pharmacokinet.; He, X., L. M. Hesse, et al. (2009). "Evidence for oxazepam as an in vivo probe of UGT2B15: oxazepam clearance is reduced by UGT2B15 D85Y polymorphism but unaffected by UGT2B17 deletion." Br J Clin Pharmacol 68(5): 721-730.; Park, W. B., P. G. Choe, et al. (2010). "Genetic factors influencing severe atazanavirassociated hyperbilirubinemia in a population with low UDP-glucuronosyltransferase 1A1∗28 allele frequency." Clin Infect Dis 51(1): 101-106.; Parmar, S., J. C. Stingl, et al. (2011). "Impact of UGT2B7 His268Tyr polymorphism on the outcome of adjuvant epirubicin treatment in breast cancer." Breast Cancer Res 13(3): R57.; Saeki, M., Y. Saito, et al. (2004). "Single nucleotide polymorphisms and haplotype frequencies of UGT2B4 and UGT2B7 in a Japanese population." Drug Metab Dispos 32(9): 1048-1054.; Sneitz, N., M. H. Court, et al. (2009). "Human UDP-glucuronosyltransferase UGT2A2: cDNA construction, expression, and functional characterization in comparison with UGT2A1 and UGT2A3." Pharmacogenet Genomics.; Sun, D., G. Chen, et al. (2006). "Characterization of tamoxifen and 4-hydroxytamoxifen glucuronidation by human UGT1A4 variants." Breast Cancer Res 8(4): R50.; Swanson, C., D. Mellstrom, et al. (2007). "The uridine diphosphate glucuronosyltransferase 2B15 D85Y and 2B17 deletion polymorphisms predict the glucuronidation pattern of androgens and fat mass in men." J Clin Endocrinol Metab 92(12): 4878-4882.; Yang, J., L. Cai, et al. (2012). "Genetic Variations and Haplotype Diversity of the UGT1 Gene Cluster in the Chinese Population." PLoS One 7(4): e33988.; Arslan, S. (2010). "Genetic polymorphisms of sulfotransferases (SULT1A1 and SULT1A2) in a Turkish population." Biochem Genet 48(11-12): 987-994.; Hirata, H., Y. Hinoda, et al. (2008). "CYP1A1, SULT1A1, and SULT1E1 polymorphisms are risk factors for endometrial cancer susceptibility." Cancer 112(9): 1964-1973.; Ji, Y., I. Moon, et al. (2007). "Human hydroxysteroid sulfotransferase SULT2B1 pharmacogenomics: gene sequence Variation and functional genomics." J Pharmacol Exp Ther 322(2): 529-540.; Ramsey, T. L., H. Y. Meltzer, et al. (2011). "Evidence for a SULT4A1 haplotype correlating with baseline psychopathology and atypical antipsychotic response." Pharmacogenomics 12(4): 471-480.; Tamaki, Y., T. Arai, et al. (2011). "Association between cancer risk and drug-metabolizing enzyme gene (CYP2A6, CYP2A13, CYP4B1, SULT1A1, GSTM1, and GSTT1) polymorphisms in cases of lung cancer in Japan." Drug Metab Pharmacokinet 26(5): 516-522.; Thomae, B. A., B. W. Eckloff, et al. (2002). "Human sulfotransferase SULT2A1 pharmacogenetics: genotype-tophenotype studies." Pharmacogenomics J 2(1): 48-56.; Thomae, B. A., O. F. Rifki, et al. (2003). "Human catecholamine sulfotransferase (SULT1A3) pharmacogenetics: functional genetic polymorphism." J Neurochem 87(4): 809-819.; Breton, C. V., H. Vora, et al. (2009). "Variation in the GST mu locus and tobacco smoke exposure as determinants of childhood lung function." Am J Respir Crit Care Med 179(7): 601-607.; Chen, Y. L., H. S. Tseng, et al. (2010). "Glutathione S-Transferase P1 (GSTP1) gene polymorphism increases age-related susceptibility to hepatocellular carcinoma." BMC Med Genet 11: 46.; Coles, B. F., F. Morel, et al. (2001). "Effect of polymorphism in the human glutathione S-transferase A1 promoter on hepatic GSTA1 and GSTA2 expression." Pharmacogenetics 11(8): 663-669.; Moyer, A. M., Z. Sun, et al. (2010). "Glutathione pathway genetic polymorphisms and lung cancer survival after platinum-based chemotherapy." Cancer Epidemiol Biomarkers Prev 19(3): 811-821.; Tetlow, N., M. Coggan, et al. (2004). "Functional polymorphism of human glutathione transferase A3: effects on xenobiotic metabolism and steroid biosynthesis." Pharmacogenetics 14(10): 657-663.; Tran, A., F. Bournerias, et al. (2008). "Serious haematological toxicity of cyclophosphamide in relation to CYP2B6, GSTA1 and GSTP1 polymorphisms." Br J Clin Pharmacol 65(2): 279-280.; White, D. L., D. Li, et al. (2008). "Genetic variants of glutathione S-transferase as possible risk factors for hepatocellular carcinoma: a HuGE systematic review and meta-analysis." Am J Epidemiol 167(4): 377-389.; Zhao, Y., M. Marotta, et al. (2009). "Linkage disequilibrium between two high-frequency deletion polymorphisms: implications for association studies involving the glutathione-S transferase (GST) genes." PLoS Genet 5(5): e1000472.; Motika, M. S., J. Zhang, et al. (2009). "Novel variants of the human flavincontaining monooxygenase 3 (FMO3) gene associated with trimethylaminuria." Mol Genet Metab 97(2): 128-135.; Voisey, J., C. D. Swagell, et al. (2011). "A novel SNP in COMT is associated with alcohol dependence but not opiate or nicotine dependence: a case control study." Behav Brain Funct 7: 51.; Fisher, M. C. and B. N. Cronstein (2009). "Metaanalysis of methylenetetrahydrofolate reductase (MTHFR) polymorphisms affecting methotrexate toxicity." J Rheumatol 36(3): 539-545.; Zhang, X. P., Z. B. Bai, et al. (2012). "Polymorphisms of dihydropyrimidine dehydrogenase gene and clinical outcomes of gastric cancer patients treated with fluorouracil-based adjuvant chemotherapy in Chinese population." Chin Med J (Engl) 125(5): 741-746.].

Es gibt auch zahlreiche Beispiele für polymorphe Transporter und/oder Rezeptoren und/oder andere Proteine.

Beispiele für polymorphe Transporter sind:
ABCA1, ABCA2, ABCA3, ABCA4, ABCA7, ABCA8, ABCA12, ABCA13, ABCB1, ABCB2, ABCB4, ABCB5, ABCB7, ABCB8, ABCB9, ABCB10, ABCB11, ABCC1, ABCC2, ABCC3, ABCC4, ABCC5, ABCC6, ABCC8, ABCC9, ABCC10, ABCC11, ABCD1, ABCD2, ABCD3, ABCD4, ABCe1, ABCF1, ABCG1, ABCG2, ABCG4, ABCG5, ABCG8, OAT1, OAT2, OAT3, OAT4, URAT5, OATP1A2, OATP1B1, OATP1B3, OATP1C1, OATP1B1, OCT1, OCT2, OCT3, OCTN1, OCTN2, SLC22A16
[Akiyama, Y., K. I. Fujita, et al. (2011). "Association of ABCC2 genotype with efficacy of first-line FOLFIRI in Japanese patients with advanced colorectal cancer." Drug Metab Pharmacokinet.; Fukao, M., K. Ishida, et al. (2011). "Effect of genetic polymorphisms of SLC28A1, ABCG2, and ABCC4 on bioavailability of mizoribine in healthy Japanese males." Drug Metab Pharmacokinet 26(5): 538-543.; Garcia-Donas, J., E. Esteban, et al. (2011). "Single nucleotide polymorphism associations with response and toxic effects in patients with advanced renal-cell carcinoma treated with first-line sunitinib: a multicentre, observational, prospective study." Lancet Oncol 12(12): 1143-1150.; Hollingworth, P., D. Harold, et al. (2011). "Common variants at ABCA7, MS4A6A/MS4A4E, EPHA1, CD33 and CD2AP are associated with Alzheimer's disease." Nat Genet 43(5): 429-435.; Iida, A., S. Saito, et al. (2002). "Catalog of 605 single-nucleotide polymorphisms (SNPs) among 13 genes encoding human ATP-binding cassette transporters: ABCA4, ABCA7, ABCA8, ABCD1, ABCD3, ABCD4, ABCE1, ABCF1, ABCG1, ABCG2, ABCG4, ABCG5, and ABCG8." J Hum Genet 47(6): 285-310.; Karadeniz, M., M. Erdogan, et al. (2011). "Effect Of G2706A and G1051A polymorphisms of the ABCA1 gene on the lipid, oxidative stress and homocystein levels in Turkish patients with polycystic ovary syndrome." Lipids Health Dis 10: 193.; Kelsell, D. P., E. E. Norgett, et al. (2005). "Mutations in ABCA12 underlie the severe congenital skin disease harlequin ichthyosis." Am J Hum Genet 76(5): 794-803.; Knight, H. M., B. S. Pickard, et al. (2009). "A cytogenetic abnormality and rare coding variants identify ABCA13 as a candidate gene in schizophrenia, bipolar disorder, and depression." Am J Hum Genet 85(6): 833-846.; Kwan, P., V. Wong, et al. (2011). "Gene-wide tagging study of the association between ABCC2, ABCC5 and ABCG2 genetic polymorphisms and multidrug resistance in epilepsy." Pharmacogenomics 12(3): 319-325.; Liptrott, N. J., S. Pushpakom, et al. (2012). "Association of ABCC10 polymorphisms with nevirapine plasma concentrations in the German Competence Network for HIV/AIDS." Pharmacogenet Genomics 22(1): 10-19.; Maia-Lopes, S., J. Aguirre-Lamban, et al. (2009). "ABCA4 mutations in Portuguese Stargardt patients: identification of new mutations and their phenotypic analysis." Mol Vis 15: 584-591.; Matsukawa, T., M. Asheuer, et al. (2011). "Identification of novel SNPs of ABCD1, ABCD2, ABCD3, and ABCD4 genes in patients with X-linked adrenoleukodystrophy (ALD) based on comprehensive resequencing and association studies with ALD phenotypes." Neurogenetics 12(1): 41-50.; Minster, R. L., S. T. DeKosky, et al. (2009). "No association of DAPK1 and ABCA2 SNPs on chromosome 9 with Alzheimer's disease." Neurobiol Aging 30(11): 1890-1891.; Moitra, K., M. Scally, et al. (2011). "Molecular evolutionary analysis of ABCB5: the ancestral gene is a full transporter with potentially deleterious single nucleotide polymorphisms." PLoS One 6(1): e16318.; Pietrzak-Nowacka, M., K. Safranow, et al. (2012). "Association of C49620T ABCC8 polymorphism with anthropometric and metabolic parameters in patients with autosomal dominant polycystic kidney disease: a preliminary study." Nefrologia 32(2): 153-159.; Saito, S., A. Iida, et al. (2002). "Identification of 779 genetic variations in eight genes encoding members of the ATP-binding cassette, subfamily C (ABCC/MRP/CFTR." J Hum Genet 47(4): 147-171.; Saito, S., A. Iida, et al. (2002). "Three hundred twenty-six genetic variations in genes encoding nine members of ATP-binding cassette, subfamily B (ABCB/MDR/TAP), in the Japanese population." J Hum Genet 47(1): 38-50.; Sasaki, T., T. Hirota, et al. (2011). "Systematic screening of human ABCC3 polymorphisms and their effects on MRP3 expression and function." Drug Metab Pharmacokinet 26(4): 374-386.; Schulz, V., D. Hendig, et al. (2005). "Analysis of sequence variations in the ABCC6 gene among patients with abdominal aortic aneurysm and pseudoxanthoma elasticum." J Vasc Res 42(5): 424-432.; Shulenin, S., L. M. Nogee, et al. (2004). "ABCA3 gene mutations in newborns with fatal surfactant deficiency." N Engl J Med 350(13): 1296-1303.; Toyoda, Y. and T. Ishikawa (2010). "Pharmacogenomics of human ABC transporter ABCC11 (MRP8): potential risk of breast cancer and chemotherapy failure." Anticancer Agents Med Chem 10(8): 617-624.; Wasmuth, H. E., A. Glantz, et al. (2007). "Intrahepatic cholestasis of pregnancy: the severe form is associated with common variants of the hepatobiliary phospholipid transporter ABCB4 gene." Gut 56(2): 265-270.; Yin, J. Y., Q. Huang, et al. (2009). "Characterization and analyses of multidrug resistanceassociated protein 1 (MRP1/ABCC1) polymorphisms in Chinese population." Pharmacogenet Genomics 19(3): 206-216.; Yu, X., H. Xie, et al. (2011). "Association of MDR1 gene SNPs and haplotypes with the tacrolimus dose requirements in Han Chinese liver transplant recipients." PLoS One 6(11): e25933.; Lee, W., H. Glaeser, et al. (2005). "Polymorphisms in human organic anion-transporting polypeptide 1A2 (OATP1A2): implications for altered drug disposition and central nervous system drug entry." J Biol Chem 280(10): 9610-9617.; Mougey, E. B., H. Feng, et al. (2009). "Absorption of montelukast is transporter mediated: a common variant of OATP2B1 is associated with reduced plasma concentrations and poor response." Pharmacogenet Genomics 19(2): 129-138.; Trdan Lu 353 In, T., B. Stieger, et al. (2012). "Organic anion transporting polypeptides OATP1B1 and OATP1B3 and their genetic variants influence the pharmacokinetics and pharmacodynamics of raloxifene." J Transl Med 10(1): 76.; van der Deure, W. M., P. S. Hansen, et al. (2008). "Thyroid hormone transport and metabolism by organic anion transporter 1C1 and consequences of genetic variation." Endocrinology 149(10): 5307-5314.; Vormfelde, S. V., M. Schirmer, et al. (2006). "Torsemide renal clearance and genetic variation in luminal and basolateral organic anion transporters." Br J Clin Pharmacol 62(3): 323-335.; Xu, G., V. Bhatnagar, et al. (2005). "Analyses of coding region polymorphisms in apical and basolateral human organic anion transporter (OAT) genes [OAT1 (NKT), OAT2, OAT3, OAT4, URAT (RST)]." Kidney Int 68(4): 1491-1499.; Becker, M. L., L. E. Visser, et al. (2011). "OCT1 polymorphism is associated with response and survival time in anti-Parkinsonian drug users." Neurogenetics 12(1): 79-82.,Lal, S., Z. W. Wong, et al. (2007). "Novel SLC22A16 polymorphisms and influence on doxorubicin pharmacokinetics in Asian breast cancer patients." Pharmacogenomics 8(6): 567-575.,Park, T. J., J. H. Kim, et al. (2011). "Possible association of SLC22A2 polymorphisms with aspirin-intolerant asthma." Int Arch Allergy Immunol 155(4): 395-402.,Sakata, T., N. Anzai, et al. (2010). "Functional analysis of human organic cation transporter OCT3 (SLC22A3) polymorphisms." J Pharmacol Sci 113(3): 263-266.,Tahara, H., S. W. Yee, et al. (2009). "Functional genetic variation in the basal promoter of the organic cation/carnitine transporters OCTN1 (SLC22A4) and OCTN2 (SLC22A5)." J Pharmacol Exp Ther 329(1): 262-271.]

Besonders hervorzuheben sind hierbei: ABCB1 (p-Glykoprotein), ABCC1 (MRP1), ABCG2 (BCRP), OATP1B1, OAT3, OCT1, OCT2, OCT3, SLC22A16.

In der Pharmakotherapie können derartige Unterschiede in der Enzymaktivität bzw. Enzymmenge einen dramatischen Einfluss auf den Behandlungserfolg haben, da sie unmittelbar die Pharmakokinetik - und hier insbesondere die Exposition - der Substanzen, welche Substrate für ein oder mehrere polymorphe Enzyme sind, sowie des/der vom polymorphen Enzym gebildeten Metaboliten beeinflussen. Dasselbe gilt für derartige Unterschiede in der Proteinaktivität bzw. Proteinmenge, da auch Rezeptoren, Transporter oder andere Proteine unmittelbar die Pharmakokinetik - und hier insbesondere die Exposition - der Substanzen, welche Substrate für ein oder mehrere polymorphe Proteine sind, beeinflussen können. Zusätzlich kann sich hier auch ein direkter Effekt auf die Pharmakodynamik ergeben, wenn diese Proteine in den Wirkungsmechanismus involviert sind.

Es bestand daher der Bedarf nach einer verbesserten Pharmakotherapie bei der Nutzung von Wirkstoffen, deren Wirkung von der Menge bzw. der Aktivität von exprimierten und/oder inhibierten/induzierten Proteinvarianten, Enzymvarianten, Rezeptorvarianten oder Transportervarianten abhängig ist, wobei diese Pharmakotherapie die o. g. Variationen kompensiert.

Die vorliegende Offenbarung basiert auf einem neuartigen Formulierungskonzept insbesondere in Form einer Fixed·Dose·Combination (FDC), bei dem vorab bekannte individuelle Unterschiede in der Aktivität eines relevanten Proteins bei der Dosierung von zwei oder mehreren pharmakologisch aktiven Substanzen, von denen eine oder mehrere Metabolit der anderen Substanz sind, berücksichtigt werden, um einen optimalen Behandlungserfolg zu gewährleisten. Das neuartige Formulierungskonzept beruht auf einer Kompensation der unterschiedlichen Exposition der Muttersubstanz und einem oder mehreren aktiven Metaboliten durch eine gezielte, individuell an den Genotyp bzw. Phänotyp angepasste Dosierung der Kombination aus Muttersubstanz und einem/mehreren Metaboliten. Das pharmakokinetische Ziel ist die Schaffung einer "Bioäquivalenz"-ähnlichen Situation im Steady-State (d. h. nach wiederholter Einnahme), d. h. einer Übereinstimmung der Plasma-Konzentrationsverläufe der beteiligten Substanzen innerhalb eines vordefinierten Rahmens (hierfür können z. B. die in anderem Zusammenhang gängigen Kriterien herangezogen werden, siehe dazu "Stand der Technik") in Bezug auf eine Referenzpopulation, die aus dem speziellen Kontext heraus zu definieren ist.

Zur Untersuchung des erfindungsgemäßen Formulierungskonzepts wurde die Pharmakotherapie mit Tamoxifen als Beispiel ausgesucht, ohne das Konzept auf dieses Beispiel zu begrenzen.

Die vorliegende Erfindung beruht auf diesem Beispiel und ist in den Ansprüchen definiert.

Im Fall eines CYP2D6 Polymorphismus wäre beispielsweise eine Population bestehend aus Extensive Metabolizern (EMs) eine sinnvolle Referenzpopulation, da dieser Phänotyp den Wildtyp darstellt und in vielen geographischen Regionen der am weitesten verbreitete ist [Sistonen, J., A. Sajantila, et al. (2007). "CYP2D6 worldwide genetic variation shows high frequency of altered activity variants and no continental structure." Pharmacogenet Genomics 17(2): 93-101.]. Am Beispiel eines bekannten Krebsmedikamentes, Tamoxifen, soll das Problem von Genotyp- bzw. Phänotyp-abhängiger Exposition aktiver Metaboliten erläutert werden ohne sich darauf zu begrenzen.

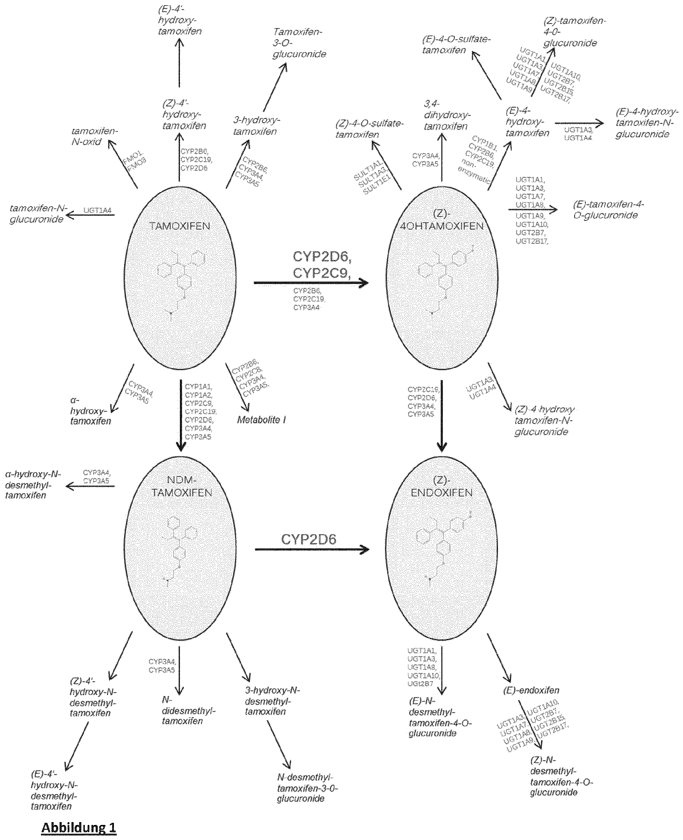

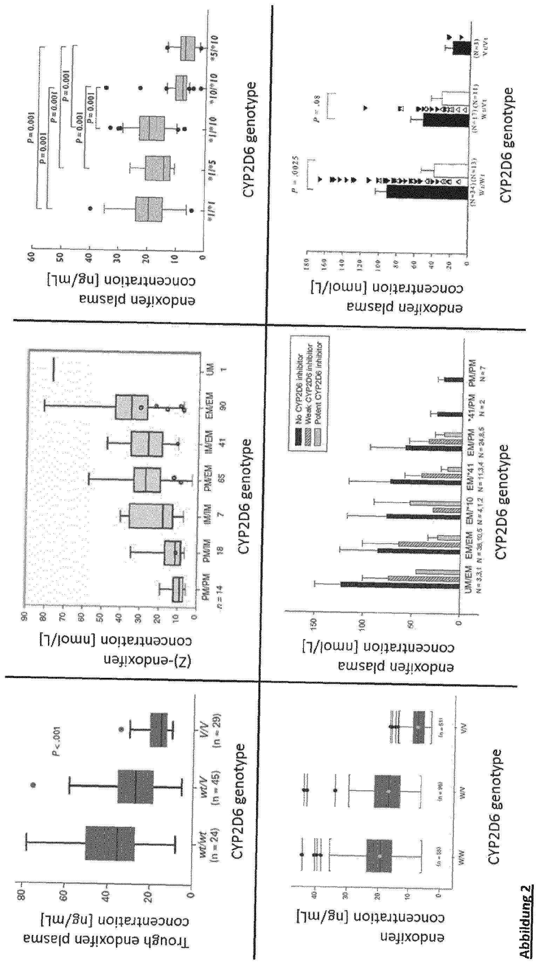

Tamoxifen ist ein lange bekannter Arzneistoff, der zur Behandlung von Östrogenrezeptorpositivem (ER+) Brustkrebs eingesetzt wird. Die Muttersubstanz unterliegt dabei einem komplexen Metabolisierungsschema, welches in **Abbildung 1** dargestellt ist. Tamoxifen wird im menschlichen Körper (neben anderen) in drei aktive Metaboliten (N-Desmethyltamoxifen, 4-Hydroxytamoxifen, Endoxifen) umgewandelt. Unter den aktiven Metaboliten ist insbesondere Endoxifen, ein sekundärer Metabolit von Tamoxifen, von Bedeutung, da die Bildung von Endoxifen zu einem großen Prozentanteil über das polymorphe CYP2D6 katalysiert wird. Dies hat zur Folge, dass die Endoxifen-Konzentration im Blut einer Brustkrebspatientin von deren CYP2D6 Genotyp bzw. Phänotyp abhängt. Bei einem CYP2D6 PM ist eine CYP2D6 Aktivität praktisch nicht vorhanden und die Konzentration des aktiven Metaboliten Endoxifen ist folglich sehr gering [Murdter, T. E., W. Schroth, et al. (2011). "Activity levels of tamoxifen metabolites at the estrogen receptor and the impact of genetic polymorphisms of phase I and II enzymes on their concentration levels in plasma." Clin Pharmacol Ther 89(5): 708-717.; Jin, Y., Z. Desta, et al. (2005). "CYP2D6 Genotype, Antidepressant Use, and Tamoxifen Metabolism During Adjuvant Breast Cancer Treatment." Journal of the National Cancer Institute 97(1): 30-39.; Gjerde, J., M. Hauglid, et al. (2008). "Effects of CYP2D6 and SULT1A1 genotypes including SULT1A1 gene copy number on tamoxifen metabolism." Ann Oncol 19(1): 56-61.; Borges, S., Z. Desta, et al. (2006). "Quantitative effect of CYP2D6 genotype and inhibitors on tamoxifen metabolism: implication for optimization of breast cancer treatment." Clin Pharmacol Ther 80(1): 61-74.; Madlensky, L., L. Natarajan, et al. (2011). "Tamoxifen metabolite concentrations, CYP2D6 genotype, and breast cancer outcomes." Clin Pharmacol Ther 89(5): 718-725.; Lim, J. S., X. A. Chen, et al. (2011). "Impact of CYP2D6, CYP3A5, CYP2C9 and CYP2C19 polymorphisms on tamoxifen pharmacokinetics in Asian breast cancer patients." Br J Clin Pharmacol 71(5): 737-750.; Lim, H. S., H. Ju Lee, et al. (2007). "Clinical implications of CYP2D6 genotypes predictive of tamoxifen pharmacokinetics in metastatic breast cancer." J Clin Oncol 25(25): 3837-3845.; Kiyotani, K., T. Mushiroda, et al. (2010). "Significant effect of polymorphisms in CYP2D6 and ABCC2 on clinical outcomes of adjuvant tamoxifen therapy for breast cancer patients." J Clin Oncol 28(8): 1287-1293.; Irvin, W. J., Jr., C. M. Walko, et al. (2011). "Genotype-Guided Tamoxifen Dosing Increases Active Metabolite Exposure in Women With Reduced CYP2D6 Metabolism: A Multicenter Study." J Clin Oncol 29(24): 3232-3239.]. Bei einem CYP2D6 IM liegt die Endoxifen-Konzentration ebenfalls noch deutlich unter dem Level, der bei einem EM oder dem (bei Europäern relativ seltenen) UM-Phänotyp zu beobachten ist. Eine Studie zeigte hierbei auch einen deutlichen Gen-Dosis-Effekt zwischen CYP2D6 EM, IM, und PM Geno- bzw. Phänotypen und ihren jeweiligen Steady-State Endoxifenkonzentrationen [Jin, Y., Z. Desta, et al. (2005). "CYP2D6 Genotype, Antidepressant Use, and Tamoxifen Metabolism During Adjuvant Breast Cancer Treatment." Journal of the National Cancer Institute 97(1): 30-39]. Die Genotyp-bzw. Phänotyp-abhängigen Expositionen von Endoxifen sind beispielhaft in **Abbildung 2** gezeigt.

Innerhalb einer Population von BrustkrebspatientInnen hängt also die Exposition von Endoxifen von der Häufigkeitsverteilung der unterschiedlichen CYP2D6 Genotypen bzw. Phänotypen ab. Diese Häufigkeitsverteilung ist regional bzw. ethnisch-bedingt stark unterschiedlich [Bernard, S., K. A. Neville, et al. (2006). "Interethnic differences in genetic polymorphisms of CYP2D6 in the U.S. population: clinical implications." Oncologist 11(2): 126-135.; Bradford, L. D. (2002). "CYP2D6 allele frequency in European Caucasians, Asians, Africans and their descendants." Pharmacogenomics 3(2): 229-243.; Sachse, C., J. Brockmoller, et al. (1997). "Cytochrome P450 2D6 variants in a Caucasian population: allele frequencies and phenotypic consequences." Am J Hum Genet 60(2): 284-295.; Sistonen, J., A. Sajantila, et al. (2007). "CYP2D6 worldwide genetic Variation shows high frequency of altered activity variants and no continental structure." Pharmacogenet Genomics 17(2): 93-101.]. Bei Europäern ist der EM der vorherrschende Genotyp [Sistonen, J., A. Sajantila, et al. (2007). "CYP2D6 worldwide genetic variation shows high frequency of altered activity variants and no continental structure." Pharmacogenet Genomics 17(2): 93-101.].

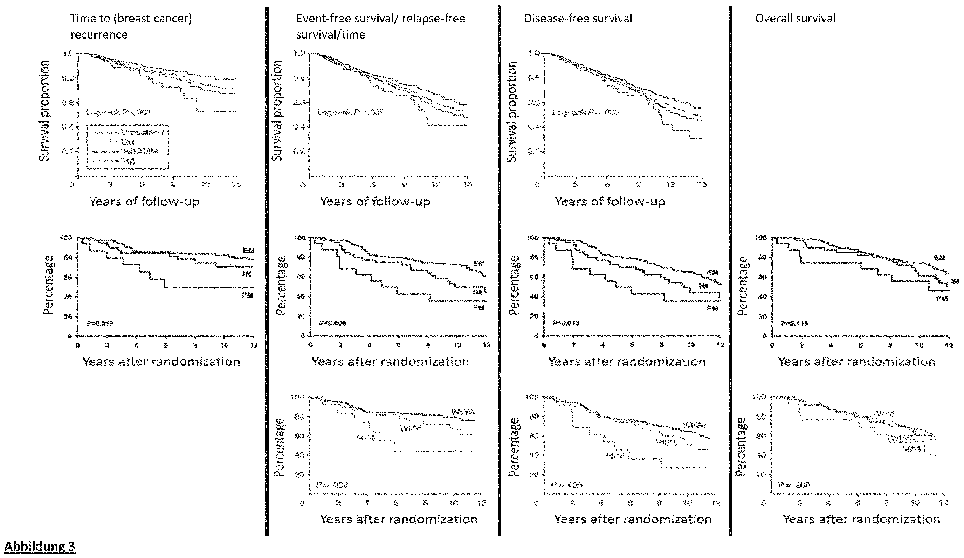

Nun gibt es eine Reihe von Studien die belegen, dass der Therapieerfolg von Tamoxifen von dem CYP2D6 Genotyp bzw. Phänotyp abhängt [Bijl, M., R. van Schaik, et al. (2009). "The CYP2D6∗4 polymorphism affects breast cancer survival in tamoxifen users." Breast Cancer Res Treat 118(1): 125-130.; Bonanni, B., D. Macis, et al. (2006). "Polymorphism in the CYP2D6 Tamoxifen-Metabolizing Gene Influences Clinical Effect but Not Hot Flashes: Data From the Italian Tamoxifen Trial." Journal of Clinical Oncology 24(22): 3708-3709.; Brauch, H., W. Schroth, et al. (2008). "Clinical Relevance of CYP2D6 Genetics for Tamoxifen Response in Breast Cancer." Breast Care (Basel) 3(1): 43-50.; Brauch, H. B., W. Schroth, et al. (2011). "CYP2D6 and Tamoxifen: Awaiting the Denouement." Journal of Clinical Oncology 29(34): 4589-4590.; Goetz, M. P., A. Kamal, et al. (2008). "Tamoxifen pharmacogenomics: the role of CYP2D6 as a predictor of drug response." Clin Pharmacol Ther 83(1): 160-166.; Goetz, M. P., S. K. Knox, et al. (2007). "The impact of cytochrome P450 2D6 metabolism in women receiving adjuvant tamoxifen." Breast Cancer Res Treat 101(1): 113-121.; Goetz, M. P., J. M. Rae, et al. (2005). "Pharmacogenetics of tamoxifen biotransformation is associated with clinical outcomes of efficacy and hot flashes." J Clin Oncol 23(36): 9312-9318.; Ingelman-Sundberg, M., S. C. Sim, et al. (2007). "Influence of cytochrome P450 polymorphisms on drug therapies: pharmacogenetic, pharmacoepigenetic and clinical aspects." Pharmacol Ther 116(3): 496-526.; Newman, W. G., K. D. Hadfield, et al. (2008). "Impaired tamoxifen metabolism reduces survival in familial breast cancer patients." Clin Cancer Res 14(18): 5913-5918.; Schroth, W., L. Antoniadou, et al. (2007). "Breast cancer treatment outcome with adjuvant tamoxifen relative to patient CYP2D6 and CYP2C19 genotypes." J Clin Oncol 25(33): 5187-5193.; Schroth, W., M. P. Goetz, et al. (2009). "Association between CYP2D6 polymorphisms and outcomes among women with early stage breast cancer treated with tamoxifen." JAMA 302(13): 1429-1436; Goetz, M.P., et al., CYP2D6 metabolism and patient outcome in the Austrian Breast and Colorectal Cancer Study Group trial (ABCSG) 8. Clin Cancer Res, 2013. 19(2): p. 500-7.; Brauch, H., et al., Tamoxifen Use in Postmenopausal Breast Cancer: CYP2D6 Matters. J Clin Oncol, 2012.]. PMs profitieren diesen Studien zu Folge deutlich weniger von einer Tamoxifentherapie als IMs, und diese wiederum weniger als EMs oder UMs, was sich beispielsweise in publizierten Rezidiv-freien Überlebenskurven (sog. Kaplan-Meier-Plots) wiederspiegelt. Beispiele solcher publizierten Plots sind in **Abbildung 3** gezeigt. Diese Studienergebnisse wurden in der Vergangenheit so interpretiert, dass die Hauptwirkung bei der Brustkrebstherapie mit Tamoxifen von dessen Metaboliten Endoxifen herrührt (Tamoxifen wird gelegentlich in der Literatur auch als "Prodrug" bezeichnet [Goetz, M. P., A. Kamal, et al. (2008). "Tamoxifen pharmacogenomics: the role of CYP2D6 as a predictor of drug response." Clin Pharmacol Ther 83(1): 160-166.]). Es wird aktuell auch in Fachkreisen der Vorschlag diskutiert, ob man nicht direkt Endoxifen anstelle von Tamoxifen verabreichen solle, und es wurden erste Studien publiziert, die eine Zulassung von reinem Endoxifen als Mittel zur Brustkrebstherapie zum Ziel haben [Ahmad, A., S. M. Ali, et al. (2010). "Orally administered endoxifen is a new therapeutic agent for breast cancer." Breast Cancer Res Treat 122(2): 579-584.; Ahmad, A., S. Shahabuddin, et al. (2010). "Endoxifen, a new cornerstone of breast cancer therapy: demonstration of safety, tolerability, and systemic bioavailability in healthy human subjects." Clin Pharmacol Ther 88(6): 814-817.].
Ebenso gibt es seit einiger Zeit Diskussionen in der Fachwelt [de Graan, A. J., S. F. Teunissen, et al. (2011). "Dextromethorphan as a phenotyping test to predict endoxifen exposure in patients on tamoxifen treatment." J Clin Oncol 29(24): 3240-3246.; Irvin, W. J., Jr., C. M. Walko, et al. (2011). "Genotype-Guided Tamoxifen Dosing Increases Active Metabolite Exposure in Women With Reduced CYP2D6 Metabolism: A Multicenter Study." J Clin Oncol 29(24): 3232-3239.; Brauch, H., W. Schroth, et al. (2008). "Clinical Relevance of CYP2D6 Genetics for Tamoxifen Response in Breast Cancer." Breast Care (Basel) 3(1): 43-50.; Lim, J. S., X. A. Chen, et al. (2011). "Impact of CYP2D6, CYP3A5, CYP2C9 and CYP2C19 polymorphisms on tamoxifen pharmacokinetics in Asian breast cancer patients." Br J Clin Pharmacol 71(5): 737-750.], ob man Patientinnen nicht vor einer Tamoxifen-Behandlung genotypisieren bzw. phänotypisieren sollte, um die Verabreichung auf die besser profitierenden EMs und UMs einzuschränken (so dass Patientinnen mit CYP2D6 Genotyp bzw. Phänotyp PM und IM ohne diese an sich wichtige Behandlungsoption auskommen müssten). Eine weitere derzeit diskutierte Therapiestrategie ist die genotyp-bzw. phänotyp-basierte Dosissteigerung von Tamoxifen, um in Patientinnen vom CYP2D6 IM und PM Phänotyp ähnliche Endoxifenkonzentrationen zu erreichen, wie sie in CYP2D6 EM Patientinnen unter einer normalen Tamoxifentherapie erreicht werden. Eine Studie zeigt hierbei, dass dieser Ansatz für die CYP2D6 IM Patientinnen eventuell eine Lösung darstellen könnte, für Patientinnen vom CYP2D6 PM Phänotyp wurden jedoch definitiv keine vergleichbaren Konzentrationen von Endoxifen erreicht. Diese Option ist für Patientinnen vom CYP2D6 PM Phänotyp folglich nicht denkbar [Irvin, W. J., Jr., C. M. Walko, et al. (2011). "Genotype-Guided Tamoxifen Dosing Increases Active Metabolite Exposure in Women With Reduced CYP2D6 Metabolism: A Multicenter Study." J Clin Oncol 29(24): 3232-3239.].
Nach dem wissenschaftlichen Stand ist davon auszugehen, dass die positive Wirkung von Tamoxifen bei ER+ Brustkrebs auf die Kombination der aktiven Komponenten zurückzuführen ist. Ohne Zweifel hat Tamoxifen selber eine antiöstrogene (und damit krebshemmende) Wirkung, ebenso wie die beiden primären Metaboliten 4-Hydroxytamoxifen und N-Desmethyltamoxifen, welche bei einer Gabe von Endoxifen nicht im Plasma der PatientIn zirkulieren würden, und man muss davon ausgehen, dass die volle Wirkung einer Tamoxifentherapie nur durch das Zusammenspiel der Muttersubstanz und ihrer aktiven Metaboliten erzielt wird [V.C. Craig, Long-Term Tamoxifen Treatment for Breast Cancer, S. 32, Allen, K. E., E. R. Clark, et al. (1980). "Evidence for the metabolic activation of nonsteroidal antioestrogens: a study of structure-activity relationships." Br J Pharmacol 71(1): 83-91.; Kemp, J. V., H. K. Adam, et al. (1983). "Identification and biological activity of tamoxifen metabolites in human serum." Biochem Pharmacol 32(13): 2045-2052.]. Folglich ist es zweifelhaft, ob eine alleinige Endoxifentherapie eine sinnvolle Alternative zur Tamoxifentherapie darstellen kann, es ist vielmehr davon auszugehen, dass eine reine Endoxifen-Gabe keine geeignete Maßnahme gegen die CYP2D6-Abhängigkeit der Tamoxifentherapie beim Östrogenrezeptor-positiven Brustkrebs darstellt.

Der wissenschaftliche Stand der Technik zur Tamoxifentherapie bei Brustkrebs ist sehr gut dokumentiert. Obwohl es sich um eine relativ alte Substanz handelt, ist die CYP2D6 Genotyp-·bzw. Phänotyp-Abhängigkeit der Tamoxifentherapie Gegenstand aktueller Forschung und lebhafter Diskussionen in der Fachwelt.

Es bestand daher der spezifische Bedarf nach einer Tamoxifen-Behandlung, die den CYP2D6 Genotyp bzw. Phänotyp berücksichtigt, die ermöglicht in Patientinnen vom CYP2D6 IM und PM Phänotyp ähnliche Endoxifenkonzentrationen zu erreichen, wie sie in CYP2D6 EM Patientinnen unter einer normalen Tamoxifentherapie erreicht werden und entsprechend auch bei den PMs und IMs zu einer erfolgversprechenden Therapie in Form einer Brustkrebsrisikominimierung führen könnte.

Zur Lösung der Aufgabe wird in der vorliegenden Erfindung eine kombinierte Verabreichung von Tamoxifen und Endoxifen in einer Arzneimittelformulierung inbesondere in einer Fixed·Dose·Combination (FDC) vorgeschlagen. Die erfindungsgemäße FDC Formulierung wird Genotyp- bzw. Phänotypspezifisch dosiert.

FDCs bestehend aus zwei oder mehreren Substanzen, welche nicht in einem Verhältnis wie Muttersubstanz und Metabolit zueinander stehen, sind nach dem Stand der Technik bekannt und werden z. B. in der HIV Therapie, Diabetes-Typ-2-Therapie, Bluthochdrucktherapie, Therapie der Hyperlipidämie oder bei der Therapie von Malaria und Tuberkulose [Anvikar, A. R., B. Sharma, et al. (2012). "Artesunate-amodiaquine fixed dose combination for the treatment of Plasmodium falciparum malaria in India." Malar J 11(1): 97. Ayede, I. A., A. G. Falade, et al. (2010). "An open randomized clinical trial in comparing two artesunate-based combination treatments on Plasmodium falciparum malaria in Nigerian children: artesunate/sulphamethoxypyrazine/pyrimethamine (fixed dose over 24 hours) versus artesunate/amodiaquine (fixed dose over 48 hours)." Malar J 9: 378.,Bramlage, P., W. P. Wolf, et al. (2010). "Effectiveness and tolerability of a fixed-dose combination of olmesartan and amlodipine in clinical practice." Vasc Health Risk Manag 6: 803-811.,Gadzhanova, S., M. Gillies, et al. (2011). "Fixed dose combination diabetes medicines - usage in the Australian veteran population." Aust Fam Physician 40(10): 811-815.,Honda, M., M. Ishisaka, et al. (2011). "Open-label randomized multicenter selection study of once daily antiretroviral treatment regimen comparing ritonavir-boosted atazanavir to efavirenz with fixed-dose abacavir and lamivudine." Intern Med 50(7): 699-705.,Kauf, T. L., K. L. Davis, et al. (2012). "Spillover adherence effects of fixed-dose combination HIV therapy." Patient Prefer Adherence 6: 155-164.,Kim, S. H., K. H. Ryu, et al. (2011). "Efficacy of fixed-dose amlodipine and losartan combination compared with amlodipine monotherapy in stage 2 hypertension: a randomized, double blind, multicenter study." BMC Res Notes 4: 461.,Mathew, J. L. (2009). "Fixed dose drug combination for treatment of tuberculosis." Indian Pediatr 46(10): 877-880.,Mengden, T., R. Hubner, et al. (2011). "Office and ambulatory blood pressure control with a fixed-dose combination of candesartan and hydrochlorothiazide in previously uncontrolled hypertensive patients: results of CHILI CU Soon." Vasc Health Risk Manag 7: 761-769.,Mengden, T., S. Uen, et al. (2009). "Management of hypertension with fixed dose combinations of candesartan cilexetil and hydrochlorothiazide: patient perspectives and clinical utility." Vasc Health Risk Manag 5: 1043-1058.,Okpechi, I. G., H. S. Schoeman, et al. (2011). "Achieving blood preSsure goals sTudy in uncontrolled hypeRtensive pAtients treated with a fixed-dose combination of ramipriL/hydrochlorothiazide: the ASTRAL study." Cardiovasc J Afr 22(2): 79-84.,Reynolds, J. K. (2009). "Fixed-dose combination of sitagliptin and metformin for the treatment of type 2 diabetes." Diabetes Metab Syndr Obes 2: 127-134.,Shiga, Y., S. Miura, et al. (2011). "Comparison of the efficacy and safety of single-pill fixed-dose combinations of losartan/hydrochlorothiazide and valsartan/hydrochlorothiazide in patients with hypertension (SALT-VAT study)." Intern Med 50(21): 2477-2483.] erfolgreich eingesetzt.

Die Vorteile gegenüber der getrennten Verabreichung zweier oder mehrerer Wirkstoffe liegen in der einfacheren Logistik, den reduzierten Kosten bei der Herstellung und Distribution, sowie (entscheidend im Fall von Tamoxifen/Endoxifen) einer verbesserten Compliance bei den Patienten.

Eine Fixed·Dose·Combination, insbesondere eine Genotyp bzw. Phänotyp-spezifische FDC, die eine Muttersubstanz und einen oder mehrere potenzielle Metaboliten enthält und zur Kompensation einer Genotyp·bzw. Phänotyp·bedingten Variabilität der Metabolitenkonzentration dient, ist nach dem Stand der Technik nicht bekannt. Ebenso ist eine Fixed Dose Combination, die eine Muttersubstanz und einen oder mehrere potentielle(n) Metabolite(n) enthält und zur Kompensation einer "phenotype-copying" bedingten Variabilität der Metabolitenkonzentration dient, nach dem Stand der Technik nicht bekannt. "Phenotype-copying" bedeutet hierbei, dass durch eine gleichzeitige Gabe des einen Medikamentes, welches über ein Enzym in einen/mehrere aktive(n) Metabolit(e) umgewandelt wird, und einen potenten Enzyminhibitor bzw. Enzyminduktor der diese Umwandlung hemmt bzw. induziert, der ursprüngliche Phänotyp des Patienten/der Patientin in einen anderen auf Grund der Wechselwirkung zwischen Enzym und Enzyminhibitor bzw. Enzyminduktor umgewandelt wird. Ein einleuchtendes Beispiel ist hierbei die Gabe von einem potenten CYP2D6-Inhibitor (beispielsweise Paroxetin) an einen Patienten/eine Patientin vom CYP2D6 EM Phänotyp, welcher/welche gleichzeitig Tamoxifen erhält. Durch die Wirkstoff-vermittelte (beispielsweise Paroxetin) CYP2D6-Inhibition stellt der/die ursprüngliche CYP2D6-EM Patient/Patientin faktisch einen IM oder PM dar und hat entsprechend niedrigere Endoxifenkonzentrationen, dem aktiven sekundären Metaboliten von Tamoxifen [Borges, S., Z. Desta, et al. (2006). "Quantitative effect of CYP2D6 genotype and inhibitors on tamoxifen metabolism: implication for optimization of breast cancer treatment." Clin Pharmacol Ther 80(1): 61-74.; Jin, Y., Z. Desta, et al. (2005). "CYP2D6 Genotype, Antidepressant Use, and Tamoxifen Metabolism During Adjuvant Breast Cancer Treatment." Journal of the National Cancer Institute 97(1): 30-39.,Stearns, V., M. D. Johnson, et al. (2003). "Active tamoxifen metabolite plasma concentrations after coadministration of tamoxifen and the selective serotonin reuptake inhibitor paroxetine." J Natl Cancer Inst 95(23): 1758-1764.].

Anstelle einer Brustkrebstherapie mit reinem Endoxifen ist der Ansatz einer erfindungsgemäßen kombinierten Gabe von Tamoxifen und Endoxifen in solchen Patientinnen, die nicht ausreichend Endoxifen bilden können (also CYP2D6 PMs und IMs), aufgrund der nachgewiesenen Wirksamkeit von Tamoxifen, N-Desmethyltamoxifen und 4-Hydroxytamoxifen vorteilhaft. Das Ziel einer solchen kombinierten Verabreichung sollte sein, die aufgrund des Genotyps bzw. Phänotyps reduzierte Bildung von Endoxifen durch Gabe einer entsprechenden Endoxifen-Dosis zu kompensieren und gleichzeitig gegebenenfalls die Dosis von Tamoxifen so anzupassen, dass PMs und IMs vergleichbare Plasmakonzentrationen im Steady-State von Tamoxifen, N-Desmethyltamoxifen, 4-Hydroxytamoxifen und Endoxifen wie EMs oder UMs unter einer reinen Tamoxifengabe erreichen.

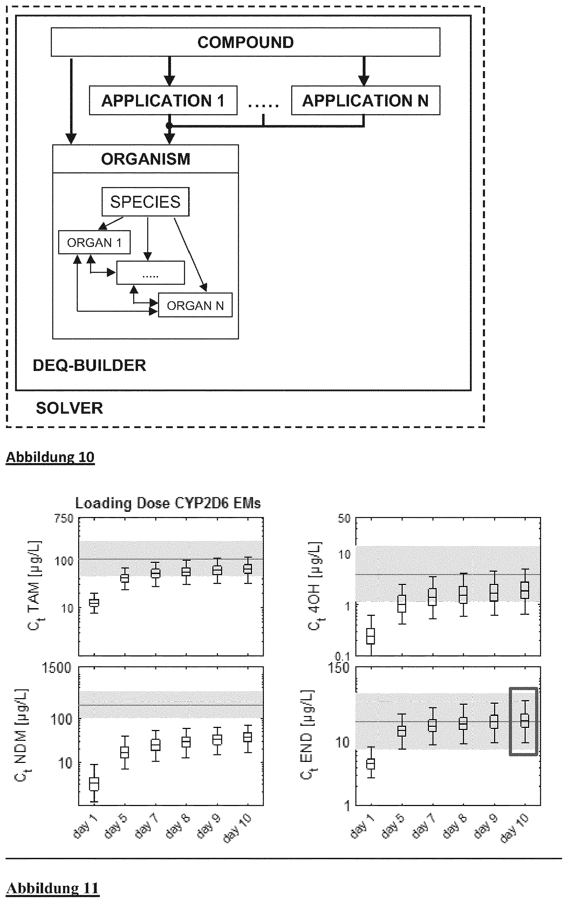

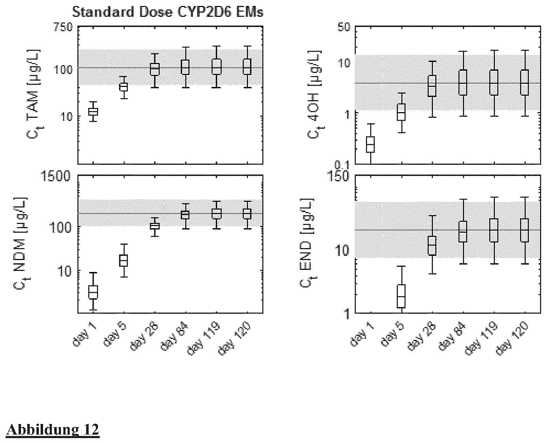

Über die voran genannten Vorteile der Tamoxifen-Endoxifen FDCs für CYP2D6 IMs und PMs hinaus kann eine Anwendung der vorgeschlagenen fixen Kombination, insbesondere aus 20 mg Tamoxifen und 3 mg Endoxifen, unter bestimmten Umständen auch in CYP2D6 EMs und IMs vorteilhaft sein. Beispielsweise lässt sich in der initialen Phase einer Tamoxifen-Therapie der Zeitraum bis zur Erreichung der gewünschten Gleichgewichtskonzentration (auch Steady-State Konzentration genannt) erheblich verkürzen. Bei der therapeutischen Standarddosierung von 20 mg Tamoxifen wird die Steady-State Konzentration von Endoxifen in einer Beispielpopulation bestehend aus europäischen Patientinnen vom CYP2D6 EM Geno- bzw. Phänotyp nach etwa 80 Tagen erreicht [Fabian C, Sternson L, El-Serafi M, Cain L, Hearne E.; Clinical pharmacology of tamoxifen in patients with breast cancer: correlation with clinical data. Cancer. 1981 Aug 15;48(4):876-82.; Jin Y, Desta Z, Stearns V, Ward B, Ho H, Lee KH, Skaar T, Storniolo AM, Li L, Araba A, Blanchard R, Nguyen A, Ullmer L, Hayden J, Lemler S, Weinshilboum RM, Rae JM, Hayes DF, Flockhart DA.; CYP2D6 genotype, antidepressant use, and tamoxifen metabolism during adjuvant breast cancer treatment. J Natl Cancer Inst. 2005 Jan 5;97(1):30-9.; Fuchs WS, Leary WP, van der Meer MJ, Gay S, Witschital K, von Nieciecki A.; Pharmacokinetics and bioavailability of tamoxifen in postmenopausal healthy women. Arzneimittelforschung. 1996 Apr;46(4):418-22.]. Wird dagegen die Tamoxifentherapie initial mit der vorgeschlagenen fixen Kombination durchgeführt, so wurde anhand des PBPK-Modells gezeigt, dass sich die wirksamen Steady-State Konzentrationen von Endoxifen deutlich schneller, nämlich bereits nach 9 Tagen einstellen, wie in den **Abbildungen** 11 und 12 gezeigt.

Die für den Beginn einer Brustkrebstherapie mit Tamoxifen gezeigten Vorteile der fixen Tamoxifen-Endoxifen-Kombination lassen sich weiterhin auch auf den in der Realität häufig auftretenden Fall der Unterbrechung der kontinuierlichen Medikamenteneinnahme (auch bezeichnet als Compliance-Mängel) übertragen. Derartige Compliance-Mängel sind bei Tamoxifen-Patientinnen bekannt und gut dokumentiert. Eine schlechte Compliance wird mit einem möglichen schlechteren Ansprechen auf eine Tamoxifen-Therapie assoziiert [Barron, T.I., et al., Early discontinuation of tamoxifen: a lesson for oncologists. Cancer, 2007. 109(5): p. 832-9.; Dezentje, V.O., et al., Effect of concomitant CYP2D6 inhibitor use and tamoxifen adherence on breast cancer recurrence in early-stage breast cancer. J Clin Oncol, 2010. 28(14): p. 2423-9.; Friese, C.R., et al., Adjuvant endocrine therapy initiation and persistence in a diverse sample of patients with breast cancer. Breast Cancer Res Treat, 2013.; Hershman, D.L., et al., Early discontinuation and nonadherence to adjuvant hormonal therapy in a cohort of 8,769 early-stage breast cancer patients. J Clin Oncol, 2010. 28(27): p. 4120-8.; McCowan, C., et al., Cohort study examining tamoxifen adherence and its relationship to mortality in women with breast cancer. Br J Cancer, 2008. 99(11): p. 1763-8.; Partridge, A.H., Nonadherence to endocrine therapy for breast cancer. Ann Oncol, 2006. 17(2): p. 183-4.; Rae, J.M., et al., Cytochrome P450 2D6 activity predicts discontinuation of tamoxifen therapy in breast cancer patients. Pharmacogenomics J, 2009. 9(4): p. 258-64.; Ruddy, K.J. and A.H. Partridge, Adherence with adjuvant hormonal therapy for breast cancer. Ann Oncol, 2009. 20(3): p. 401-2.; Ziller, V., et al., Adherence to adjuvant endocrine therapy in postmenopausal women with breast cancer. Ann Oncol, 2009. 20(3): p. 431-6.].

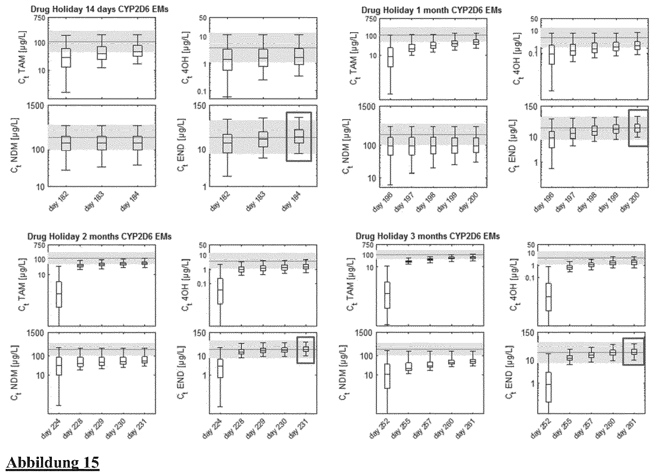

Bei einer Tamoxifen-Einnahmepause sinken die Plasmaspiegel von Tamoxifen und seinen aktiven Metaboliten (insbesondere also auch des Endoxifens) unter die therapeutisch wirksame Schwelle. Ähnlich wie bei der initialen Tamoxifentherapie kann die fixe Kombination hier ebenfalls in CYP2D6 EMs und IMs vorteilhaft eingesetzt werden, um das erneute Erreichen von wirksamen Konzentrationen zu beschleunigen, wie von den Ergebnissen der Simulationen in den **Abbildungen** 15 bis 18 dargestellt.

Ein Gegenstand der vorliegenden Offenbarung ist daher eine Arzneimitteilformulierung enthaltend eine Muttersubstanz, deren Wirkung von der Menge bzw. der Aktivität von exprimierten und/oder inhibierten/induzierten Proteinvarianten, Enzymvarianten, Rezeptorvarianten oder Transportervarianten abhängig ist, und einem oder mehreren potenziellen Metaboliten der Muttersubstanz. Insbesondere ist die Dosierung der erfindungsgemäßen Formulierung Genotyp- bzw. Phänotypisch-spezifisch definiert.

Doch eine solche kombinierte Formulierung mehrerer pharmazeutisch aktiver Substanzen ist mit Schwierigkeiten behaftet. Die Hauptschwierigkeit besteht in der Bestimmung der optimalen Endoxifen- sowie Tamoxifendosis, die für die therapeutisch wirksamen Plasmaspiegel in CYP2D6 PMs und IMs im Steady-State sorgt.

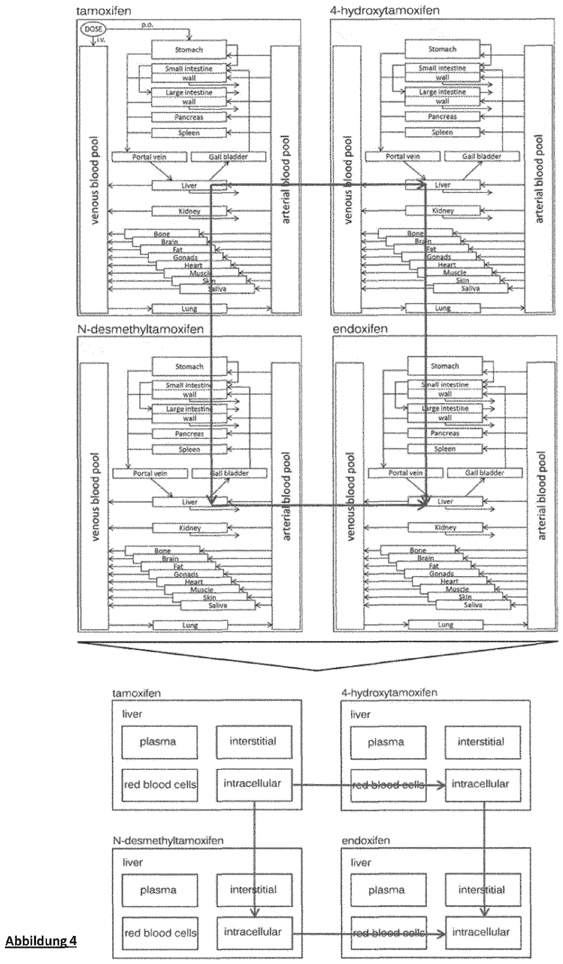

Diese weitere Aufgabe wurde bei der vorliegenden Erfindung mit Hilfe einer Methode basierend auf der Nutzung eines gekoppelten Physiologie-basierten Pharmakokinetik (PBPK) Modells für Tamoxifen, 4-Hydroxytamoxifen, N-Desmethyltamoxifen und Endoxifen gelöst. Diese Methode und das entsprechende kommerziell erhältliche Modell *PK-Sim*®/*MoBi*® ist in den Anmeldungen WO2007/147539, WO05/116854 und WO 05/033982 beschrieben, deren Lehre in der Hinsicht hiermit integriert werden, und bei der vorliegenden Erfindung zur Entwicklung einer Methode basierend auf einem gekoppelten PBPK-Modell herangezogen werden. Die Entwicklung des gekoppelten PBPK-Modells für Tamoxifen, N-Desmethyltamoxifen, 4-Hydroxytamoxifen und Endoxifen in CYP2D6 EMs und PMs ist bereits beschrieben [Dickschen, K., et al., Physiologically-based pharmacokinetic modeling of tamoxifen and its metabolites in women of different CYP2D6 phenotypes provides new insight into the tamoxifen mass balance. Frontiers in Pharmacology, 2012. 3.]. Die Methode wurde anschließend beispielhaft für die Optimierung der Tamoxifen- und Endoxifen-Dosen in CYP2D6 PMs und IMs verwendet. Dabei besteht der einzige Unterschied zwischen der publizierten CYP2D6 PM Modellparameterisierung und der hier zusätzlich präsentierten CYP2D6 IM Parameterisierung in dem verwendeten Faktor für die CYP2D6 Enzymaktivität (IM: 0.62; PM 0.015 [Coller, J. K., N. Krebsfaenger, et al. (2002). "The influence of CYP2B6, CYP2C9 and CYP2D6 genotypes on the formation of the potent antioestrogen Z-4-hydroxy-tamoxifen in human liver." Br J Clin Pharmacol 54(2): 157-167.]). In **Abbildung 4** ist eine schematische Darstellung des gekoppelten PBPK-Modells für Tamoxifen und seine drei aktiven Metaboliten (N-Desmethyltamoxifen, 4-Hydroxytamoxifen, Endoxifen) dargestellt.

Ein Gegenstand der vorliegenden Offenbarung ist auch eine Methode zur Herstellung einer Fixed·Dose·Combination Arzneitmittelformulierung umfassend eine Muttersubstanz, deren Wirkung von der Menge bzw. der Aktivität von exprimierten und/oder inhibierten/induzierten Proteinvarianten, Enzymvarianten, Rezeptorvarianten oder Transportervarianten abhängig ist, und mindestens einen Metaboliten der Muttersubstanz, mit folgenden Schritten:
a) Eingabe eines Organismus, dessen Genotyp bzw. Phänotyp, der Muttersubstanz und mindestens des Metaboliten der Muttersubstanz, eines optimalen Referenzplasmaspiegels im Steady-State für die Muttersubstanz für einen Referenzgenotyp bzw. Referenzphänotyp bei Einreichung der Muttersubstanz alleine in ein Eingabemodul,
b) Weiterleitung der Daten aus a) in ein Berechnungsmodul umfassend ein Substanzdatenmodul, ein Organismusdatenmodul, ein Genotypdatenmodul bzw. ein Phänotypdatenmodul, und ein Physiologie-basiertes pharmakokinetisches Modell, wobei das Substanzdatenmodul Daten über die physiko-chemischen und/oder biochemischen Eigenschaften der Substanz(en), das Organismusmodul Daten über die Kompartimente des Organismus, und das Genotypdatenmodul bzw. Phänotypdatenmodul Genotyp bzw. Phänotyp-spezifische Daten umfasst,
c) Automatische Selektion von Muttersubstanz und Metabolit(en)-spezifischen Daten aus dem Substanzdatenmodul,
d) Automatische Selektion von Organismus-spezifischen Daten aus dem Organismusdatenmodul anhand der Eingabe a),
e) Automatische Selektion von Genotyp-bzw. Phänotyp-spezifischen Daten aus dem Genotypdatenmodul bzw. Phänotypdatenmodul,
f) Weiterleitung der selektierten Daten aus a) bis e) in das Physiologie-basierte pharmakokinetische Modell,
g) Berechnung durch das Physiologie-basierte pharmakokinetische Modell einer optimierten Dosierung für die Muttersubstanz für den Referenzgenotyp bzw. Referenzphänotyp zur Erreichung des eingegebenen optimalen Referenzplasmaspiegels für die Muttersubstanz aus a),
h) Berechnung des Referenzplasmaspiegels im Steady-State für die Metabolite (N-Desmethyltamoxifen, 4-Hydroxytamoxifen, Endoxifen) für den Referenzgenotyp bzw. Referenzphänotyp bei Gabe der in g) berechneten Dosis der Muttersubstanz,
i) Berechnung eines aufgrund des in a) eingegebenen Genotyps bzw. Phänotyps reduzierten Plasmaspiegels des/der Metabolite(n) gegenüber des entsprechenden Referenzplasmaspiegels bei Gabe der in g) berechneten Dosis der Muttersubstanz,
j) Berechnung einer Metaboliten-Dosis sowie einer Muttersubstanz-Dosis zum kombinierten Erreichen der Referenzplasmaspiegels für den/die Metabolite(n) aus h) und des Referenzplasmaspiegels für die Muttersubstanz aus a),
k) Ausgabe der Metaboliten-Dosis sowie der Muttersubstanz-Dosis für die Fixed·Dose·Combination Arzneimitteilformulierung über ein Ausgabemodul, und / oder
l) Weiterleitung der in j) berechneten Dosis in eine automatische Vorrichtung zur Dosierung von Medikamenten.

Als automatische Vorrichtung zur Dosierung von Medikamenten sind bei der vorliegenden Erfindung Vorrichtungen zur Herstellung von Darreichungsformen wie z. B. Tabletten, Kapsel, Flüssigkeitsdosis oder Elemente davon, sowie Apparate zur Abmessung der Dosierung wie eine Waage, nach dem Stand der Technik bekannte Unit-Dose Systeme, oder eine Vorrichtung zur volumetrischen oder gravimetrischen Abmessung von Flüssigkeiten gemeint.

Optional weist das Berechnungsmodul zusätzlich ein Applikationsmodul auf, das Daten über Darreichungsformen wie beispielweise Tabletten, Kapsel, Flüssigkeitsdosis, oder Elemente davon umfasst. Diese Daten umfassen üblicherweise Freisetzungseigenschaften der Darreichungsform wie sofortige, verzögerte Freisetzung sowie differenzierte (z. B. durch schichtenartige Wirkstoffverteilung) oder gleichzeitige Freisetzung (z. B. durch gemeinsame Granulierung) für Kombinationsformulierungen. In dem Eingabemodul kann dann die Darreichungsform ausgewählt definiert werden, und die Daten über die entsprechende Darreichungsform werden aus dem Applikationsmodul automatisch selektiert und an das Physiologie-basierte pharmakokinetische Modell weitergeleitet.

Das Berechnungsmodul berechnet die optimale Medikamentendosis für die Muttersubstanz und den/die Metabolite(n) und gegebenenfalls ein optimales Dosierungsschema. Es besteht aus einer computerimplementierten Software und der zur Ausführung des Programms benötigten Hardware. Bei der Hardware handelt es sich in der Regel um einen handelsüblichen PC. Dieser ist entweder direkt mit einem Eingabegerät verbunden, wie im Falle eines Laptop-Computers mit eingebauter Tastatur oder Chipkartenlesegerät, oder dezentral aufgestellt und mit dem Eingabegerät verbunden (Server). Dabei sind prinzipiell alle gängigen Übermittlungstechniken, sowohl kabelgebundene als auch drahtlose Verfahren geeignet und denkbar. Besonders bevorzugt ist eine drahtlose Übermittlung der über das Handheld-Eingabemodul oder den Chipkartenleser eingegebenen Patienteninformationen.

Von der Software können alle zur Berechnung der optimalen Medikamentendosierung relevanten Informationen in einer oder mehreren Datenbanken verwaltet werden. In einer besonderen Ausführungsform des Verfahrens kann auch die Berechnung einer patientenindividuellen Dosis vorgenommen werden. Diese zur Berechnung der Medikamentendosis relevanten Informationen sind üblicherweise unterteilt in Organismus-, Substanz-, Genotyp bzw. Phänotyp- und bevorzugt Administration-spezifische Daten, und vorzugsweise in entsprechenden Datenmodulen automatisch abrufbar gespeichert.

In einer besonderen Ausführungsform, die für die personalisierte Medikation besonders relevant ist, werden auch physiologische (bzw. anthropometrische) Informationen, pathologische Informationen, gegebenenfalls Informationen zu zusätzlich verabreichten Medikamenten, so genannter Ko-Medikation als Patient-spezifische Daten automatisch abrufbar ebenfalls in Datenmodule gespeichert.

Zu den Substanzdaten gehören beispielsweise Lipophilie, freie Plasmafraktion, Blut-Plasma-Ratio, Partitionskoeffizienten, Permeabilität, Verteilungsvolumen, Clearance, Art der Clearance, Clearance-Proportionen, Art der Ausscheidung, Dosierungsschema, Transportersubstrat, pharmakokinetischer und/oder pharmakodynamischer Endpunkt und Nebenwirkungen.

Relevante Medikamenteninformationen sind insbesondere die empfohlene therapeutische Dosierung (nach Herstellerangaben), pharmakokinetischer und/oder pharmakodynamischer Endpunkt, Clearance (Gesamtclearance als Blut- oder Plasmaclearance in einer Referenzpopulation oder einem Referenzindividuum) und Art der Clearance (hepatisch metabolisch, biliär, renal etc.) sowie die Anteile der einzelnen Prozesse an der Gesamtclearance, kinetische Parameter von aktiven Transportern/Rezeptoren/Enzymen, sofern das Medikament und/oder sein(e) Metabolit(en) Substrat für ein oder mehrere aktive Transporter/Rezeptoren/Enzymen ist, sowie physikochemische und pharmakokinetische Informationen wie z. B. Lipophilie, ungebundene Fraktion im Plasma, Plasmaproteine, an die das Medikament und/oder sein(e) Metabolit(en) bindet , Blut/Plasma-Verteilungskoeffizient, oder Verteilungsvolumen.

Empirisches Wissen, welches z. B. durch die Recherche von Fallstudien gewonnen werden kann, kann ebenfalls zusätzlich Bestandteil der Datenbanken mit Substanz Informationen oder Informationen zu Ko-Medikamenten sein.

Relevante physiologische bzw. anthropometrische und pathophysiologische Informationen sind analog zu den individuellen Patienteninformationen beispielsweise jeweils Alter, Geschlecht, Rasse, Körpergewicht, Körpergröße, Body Mass Index, Lean Body Mass, Fat Free Body Mass, Genexpressionsdaten, Krankheiten, Allergien, Medikation, Nierenfunktion und Leberfunktion. Relevante pathophysiologische Informationen sind insbesondere Krankheiten, Allergien, Nierenfunktion und Leberfunktion.

Im Falle von Ko-Medikationen sind die entsprechenden vorgenannten Informationen über alle weiteren verabreichten Medikamente Inhalt der Datenbank zu den Ko-Medikamenten.

Die Berechnung der optimalen Dosierung und gegebenenfalls des optimalen Dosierungsschemas erfolgt anhand der Substanz-, Organismus- und Genotyp bzw. Phänotypggf. kombiniert mit den Administration-spezifischen Daten unter Verwendung eines rationalen mathematischen Modells zur Berechnung des pharmakokinetischen und pharmakodynamischen Verhaltens der zu verabreichenden Substanzen (Muttersubstanz und Metabolit(e)) basierend auf den in den Datenbanken enthaltenen Informationen. Rationale mathematische Modelle in diesem Zusammenhang können beispielsweise allometrische Skalierfunktionen oder physiologie-basierte Pharmakokinetikmodelle sein.

In einer bevorzugten Ausführungsform der Erfindung wird zur Berechnung der individuellen Dosierung ein physiologisch-basiertes pharmakokinetisch/pharmakodynamisches Simulationsmodell verwendet. Besonders bevorzugt ist das dynamisch generierte physiologisch-basierte Simulationsmodell, welches in WO2005/633982 detailliert beschrieben ist.

Ein besonderer Vorteil bei der Verwendung des physiologie-basierten Simulationsmodells aus WO2005/633982 liegt in der Möglichkeit, eine gleichzeitige Verabreichung mehrerer Medikamente und deren Wechselwirkung dynamisch zu simulieren. Dynamisch bedeutet in diesem Zusammenhang, dass bei der Wechselwirkung die Kinetiken der beiden (gegebenenfalls auch von mehreren) wechselwirkenden Substanzen berücksichtigt werden können. Dies ist gegenüber einer statischen Betrachtung, bei der z. B. ein Enzym oder ein Transporter ohne Zeitabhängigkeit ganz oder teilweise gehemmt wird, von Vorteil, da die dynamische Simulation eine Optimierung des Dosierungsschematas erlaubt. Ein mögliches Ergebnis einer solchen Optimierung des Dosierungsschemas ist beispielsweise, einen maximalen Zeitabstand von z. B. 12 Stunden (bei einmal täglicher Verabreichung) bei der Verabreichung zweier wechselwirkender Substanzen einzuhalten, um den gegenseitigen Einfluss zu minimieren.

Besonders geeignet zur Durchführung des erfindungsgemäßen Verfahrens ist die Systembiologie-Software Suite bestehend aus PK-Sim® und MoBi® der Firma Bayer Technology Services GmbH.

Prozesse wie Proteininhibition oder -induktion, sind bekanntermaßen zeitabhängig, so dass auch auf diesen Prozessen basierende Interaktionseffekte ebenfalls zeitabhängig sind. In speziellen Fällen können diese dynamischen Effekte, die sich auf einer Zeitskala von mehreren Tagen oder Wochen abspielen, die Notwendigkeit einer Dosisanpassung eines Medikaments im Therapieverlauf bedingen. Eine simple statische Betrachtung oder lediglich die Ausgabe einer Warnung an den Behandler bei zeitnaher Verabreichung sich gegenseitig beeinflussender Medikamente, wie sie nach dem Stand der Technik bekannt sind, wird solchen komplexen, dynamischen Effekten nicht gerecht.

Exemplarisch ist die erfindungsgemäße Methode in der Lage, die Plasmaspiegel im Steady-State der vier Substanzen Tamoxifen, 4-Hydroxytamoxifen, N-Desmethyltamoxifen und Endoxifen in Brustkrebspatientinnen mit unterschiedlichen CYP2D6 Genotypen bzw. Phänotypen in Abhängigkeit der Tamoxifen-Dosis zu simulieren. Durch eventuell notwendige Anpassung der Tamoxifen-Dosis und eine gleichzeitige Simulation einer Verabreichung von steigenden Endoxifen-Dosierungen lässt sich mit Hilfe des Modells die Frage nach der optimalen Dosierung der beiden Wirkstoffe in CYP2D6 IMs und PMs beantworten. In diesem speziellen Fall sind die Plasmaspiegel im Steady-State die pharmakologisch kritische Größe; der genaue Zeitverlauf der Plasmakonzentration ist hier nebensächlich. Erfindungsgemäß wird üblicherweise pro Genotyp bzw. Phänotyp eine passende Kombination der Substanzen ermittelt, die den Unterschied dieses Genotyps bzw. Phänotyps im Vergleich zur Referenz kompensiert.

Als Darreichungsform wurden kommerziell erhältliche 20 mg Tamoxifen Tablett-Formulierungen mit einer einmal täglichen Gabe als Basis angenommen, bei der keine davon formulierungsbedingt retardiert oder verlangsamt ist. Eine solche Darreichungsform ist z. B. in den Fachinformationen Nolvadex® 20 mg Filmtabletten der Firma Astra Zeneca oder Tamoxifen-ratiopharm® 10mg/20mg/30mg Tabletten der Firma Ratiopharm jeweils unter dem Abschnitt 6.1 beschrieben.

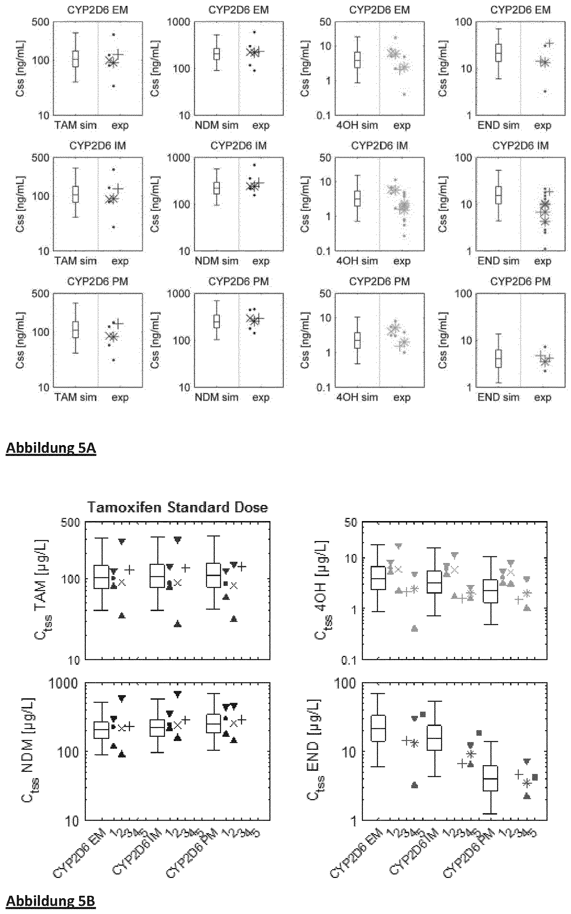

Im vorliegenden Beispiel konnte gezeigt werden, dass eine Kombination bestehend aus 20 mg Tamoxifen und 3 mg Endoxifen in CYP2D6 PMs zu vergleichbaren Plasmaspiegeln von Tamoxifen, N-Desmethyltamoxifen, 4-Hydroxytamoxifen und Endoxifen führt wie die reine Gabe von 20 mg Tamoxifen in CYP2D6 EMs. In CYP2D6 IMs stellte sich die Kombination aus 20 mg Tamoxifen und 1 mg Endoxifen als optimal heraus **(Abbildungen 5-7).**

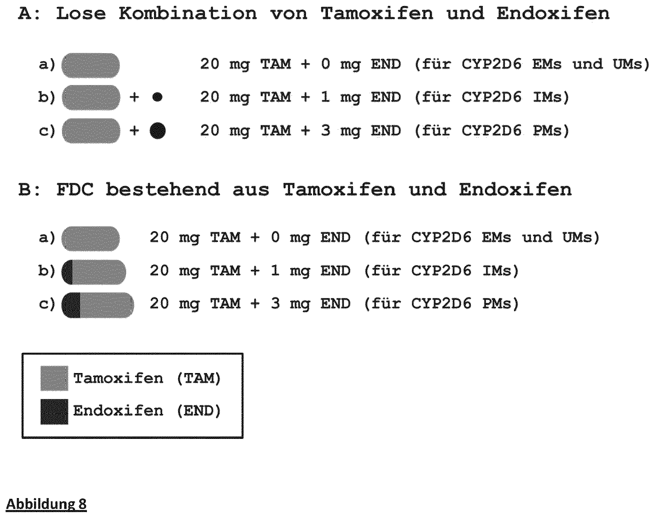

Gegenstände der vorliegenden Offenbarung sind daher:
- Eine Fixed·Dose·Combination Formulierung umfassend 15 - 25 mg Tamoxifen und 0,25 -5,0 mg Endoxifen. Insbesondere:
- eine Fixed·Dose·Combination Formulierung für CYP2D6 IM Patienten umfassend 15 - 25 mg Tamoxifen und 0,25 - 2,00 mg Endoxifen, insbesondere 18 - 22 mg Tamoxifen und 0,5 - 1,5 mg Endoxifen, besonders bevorzugt 20 mg Tamoxifen und 1,0 mg Endoxifen (**Abbildung 8** **A b**)) sowie
- eine Fixed·Dose·Combination Formulierung für CYP2D6 PM Patienten umfassend 15 - 25 mg Tamoxifen und 1,0 - 5,0 mg Endoxifen, insbesondere 18 - 22 mg Tamoxifen und 2,0 - 4,0 mg Endoxifen, insbesondere 20 mg Tamoxifen und 3,0 mg Endoxifen **(****Abbildung 8** **A c)).**

Weitere Komponenten der erfindungsgemäßen Formulierung sind aus Stand der Technik bekannt. Für die Herstellung einer erfindungsgemäßen Formulierung wird die Formulierung unter anderem aus den Fachinformationen Nolvadex® 20 mg Filmtabletten der Firma Astra Zeneca oder Tamoxifen-rationpharm® 10mg/20mg/30mg Tabletten der Firma Ratiopharm und aus Ahmad, A., et al., Endoxifen, a new cornerstone of breast cancer therapy: demonstration of safety, tolerability, and systemic bioavailability in healthy human subjects. Clin Pharmacol Ther, 2010. 88(6): p. 814-7 bzw. US 2009-0291134 A1 herangezogen.

Um höhere Endoxifen-Expositionen in Brustkrebspatientinnen zu erreichen wurde in der Vergangenheit die Tamoxifen Dosis auch versuchsweise erhöht. Anstelle der im CYP2D6 EM effektiven 20 mg Tamoxifen pro Tag wurden bis zu 40 mg Tamoxifen pro Tag als zwei Einzeldosen in CYP2D6 IMs und PMs verabreicht. Doch auch diese massive Erhöhung der Dosis der Muttersubstanz führte nicht zu den in CYP2D6 EMs beobachteten EndoxifenKonzentrationen bei einer therapeutischen Dosis von 20 mg Tamoxifen [Irvin, W.J., Jr., et al., Genotype-Guided Tamoxifen Dosing Increases Active Metabolite Exposure in Women With Reduced CYP2D6 Metabolism: A Multicenter Study. J Clin Oncol, 2011. 29(24): p. 3232-9.]. Ein besonderer Vorteil der beschriebenen Genotyp- bzw. Phänotyp-spezifischen kombinierten Gabe von Tamoxifen and Endoxifen ist daher, dass die Tamoxifen-Exposition in CYP2D6 IMs und PMs gegenüber dem CP2D6 EMs nicht stark überhöht ist (im Gegensatz zu der derzeit in der wissenschaftlichen Community propagierten Erhöhung der Tamoxifen Dosis).

Da Tamoxifen (und ebenso die propagierte nicht fixdosierte Kombinationstherapie aus Tamoxifen und Endoxifen in CYP2D6 PMs und IMs) aber über einen langen Zeitraum (typischerweise 5 Jahre) einmal täglich eingenommen werden muss, besteht eine zweite Schwierigkeit einer potenziellen Kombinationstherapie darin, eine möglichst gute Compliance zu gewährleisten. Bekanntermaßen sinkt die Compliance (und damit der Behandlungserfolg) bei einer medikamentösen Therapie, mit der Anzahl an Tabletten, die eingenommen werden müssen. Aus diesem Grunde ist es vorteilhaft Tamoxifen und Endoxifen zu einer FDC zu kombinieren. Eine FDC beinhaltet dann jeweils eine bestimmte, vom CYP2D6 Genotyp bzw. Phänotyp (PM oder IM) abhängige Dosis der beiden Wirkstoffe in Form einer einzelnen Darreichungsform (z. B. Tablette oder Kapsel).

Eine weitere bevorzugte Ausführungsform der Erfindung ist somit jeweils eine Genotyp- bzw. Phänotyp-spezifische Fixed-Dose-Combination aus Tamoxifen und Endoxifen in den bereits aufgeführten Verhältnissen.

Der am Beispiel von Tamoxifen/Endoxifen dargestellte Lösungsansatz lässt sich ohne weiteres auch auf andere Kombinationen von Muttersubstanz plus einem (oder mehreren) Metaboliten, deren Bildung durch genotypische bzw. phänotypische Besonderheiten, sowie durch das Phänomen des oben bereits erwähnten "phenotype copying" beeinflusst ist, übertragen. Insbesondere wäre für die Optimierung der Codeinwirkung eine FDC, genauer eine genotyp bzw. phänotyp-spezifische FDC von Codein und Morphin (dessen Umwandlung aus Codein ebenfalls von CYP2D6 katalysiert wird) anwendbar.

Weitere potentielle Kandidaten wären beispielsweise unter anderem: Ezlopitant, Donepezil, Clopidogrel, Cyclophosphamid, Azathioprin, Irinotecan, Leflunomid, Capecitabin, Prasugrel, Venlafaxin, Losartan, Tolterodin, Tramadol, Oxycodon, Hydrocodon, Doxorubicin, Mycophenolat-Mofetil, Estramustin, Ifosfamid, Gemcitabin, Etoposid, Terfenadin, Methotrexat.

Die beschriebene Offenbarung einer Arzneimittelformulierung, vorzugsweise einer FDC, enthaltend eine Muttersubstanz und ein oder mehrere Metaboliten kann ohne weiteres auf andere Wirkstoffkandidaten übertragen werden. Im oben ausführlich angeführten Beispiel Tamoxifen-Endoxifen besteht das Problem in der mangelnden Umwandlung von Tamoxifen zu Endoxifen bei Patienten mit einem CYP2D6 IM oder PM Phänotyp. Wie exemplarisch dargestellt, können durch die Kombination aus der Standarddosis der Muttersubstanz mit einer Genotyp- bzw. Phänotyp-spezifischen Endoxifen-Dosis für CYP2D6 IMs bzw. für CYP2D6 PMs in einer fixkombinierten Arzneistoffformulierung dieser Mangel ausgeglichen und Unterschiede im Therapieansprechen eliminiert werden.

Im Grunde genommen lässt sich das Prinzip einer Geno- bzw. Phänotyp-spezifischen Arzneimittelformulierung, vorzugsweise einer FDC, bestehend aus einer Muttersubstanz und einem oder mehreren Metaboliten, zunächst auf alle Muttersubstanzen, die durch ein polymorphes Enzym, Protein, Rezeptor oder Transporter in einen oder mehrere aktiven Metaboliten umgewandelt, und/oder gebunden und/oder transportiertwerden und/oder ihre pharmakodynamische Wirkung entfalten, übertragen.

Ein weiteres Beispiel für die Umwandlung einer Muttersubstanz in einen aktiven Metaboliten über ein polymorphes Enzym ist Clopidogrel. Clopidogrel hemmt die Blutgerinnung, nachdem es in seinen aktiven Metaboliten umgewandelt wurde, durch eine Blockade der ADP-abhängigen Thrombozytenaktivierung über den Glykoprotein-IIb/IIIa-Rezeptorkomplex. Clopidogrel wird unter anderem über das polymorphe Enzym CYP2C19 in seinen aktiven Metaboliten umgewandelt. CYP2C19 unterliegt einem ausgeprägten genetischen Polymorphismus. Ähnlich wie bei CYP2D6 findet man daher in der Bevölkerung CYP2C19 PMs. Auch hier besteht der begründete Verdacht, dass Patienten mit einem CYP2C19 PM Geno- bzw. Phänotyp nicht ausreichend von einer Therapie mit Clopidogrel profitieren könnten [Simon T, Bhatt DL, Bergougnan L, Farenc C, Pearson K, Perrin L, Vicaut E, Lacreta F, Hurbin F, Dubar M.; Genetic polymorphisms and the impact of a higher clopidogrel dose regimen on active metabolite exposure and antiplatelet response in healthy subjects., Clin Pharmacol Ther. 2011 Aug;90(2):287-95.; Lee JB, Lee KA, Lee KY.; Cytochrome P450 2C19 polymorphism is associated with reduced clopidogrel response in cerebrovascular disease. Yonsei Med J. 2011 Sep;52(5):734-8.; Kazui M, Nishiya Y, Ishizuka T, Hagihara K, Farid NA, Okazaki O, Ikeda T, Kurihara A.; Identification of the human cytochrome P450 enzymes involved in the two oxidative steps in the bioactivation of clopidogrel to its pharmacologically active metabolite. Drug Metab Dispos. 2010 Jan;38(1):92-9.; Savi P, Pereillo JM, Uzabiaga MF, Combalbert J, Picard C, Maffrand JP, Pascal M, Herbert JM.; Identification and biological activity of the active metabolite of clopidogrel. Thromb Haemost. 2000 Nov, 84(5):891-6.; Cervinski MA, Schwab MC, Lefferts JA, Lewis LD, Lebel KA, Tyropolis AM, Pflueger SM, Tsongalis GJ.; Establishment of a CYP2C19 genotyping assay for clinical use. Am J Clin Pathol. 2013 Feb, 139(2):202-7; Frelinger AL 3rd, Lee RD, Mulford DJ, Wu J, Nudurupati S, Nigam A, Brooks JK, Bhatt DL, Michelson AD.; A randomized, 2-period, crossover design study to assess the effects of dexlansoprazole, lansoprazole, esomeprazole, and omeprazole on the steady-state pharmacokinetics and pharmacodynamics of clopidogrel in healthy volunteers. J Am Coll Cardiol. 2012 Apr 3, 59(14):1304-11.; Gong IY, Crown N, Suen CM, Schwarz UI, Dresser GK, Knauer MJ, Sugiyama D, Degorter MK, Woolsey S, Tirona RG, Kim RB.; Clarifying the importance of CYP2C19 and PON1 in the mechanism of clopidogrel bioactivation and in vivo antiplatelet response. Eur Heart J. 2012 Nov, 33(22):2856-2464a.; Mega JL, Hochholzer W, Frelinger AL 3rd, Kluk MJ, Angiolillo DJ, Kereiakes DJ, Isserman S, Rogers WJ, Ruff CT, Contant C, Pencina MJ, Scirica BM, Longtine JA, Michelson AD, Sabatine MS.; Dosing clopidogrel based on CYP2C19 genotype and the effect on platelet reactivity in patients with stable cardiovascular disease. JAMA. 2011 Nov 23, 306(20):2221-8.; Zabalza M, Subirana I, Sala J, Lluis-Ganella C, Lucas G, Tomás M, Masiá R, Marrugat J, Brugada R, Elosua R.; Meta-analyses of the association between cytochrome CYP2C19 loss- and gain-of-function polymorphisms and cardiovascular outcomes in patients with coronary artery disease treated with clopidogrel. Heart. 2012 Jan;98(2):100-8., Yamamoto K, Hokimoto S, Chitose T, Morita K, Ono T, Kaikita K, Tsujita K, Abe T, Deguchi M, Miyagawa H, Saruwatari J, Sumida H, Sugiyama S, Nakagawa K, Ogawa H., Impact of CYP2C19 polymorphism on residual platelet reactivity in patients with coronary heart disease during antiplatelet therapy. J Cardiol. 2011 Mar;57(2):194-201.; Jin B, Ni HC, Shen W, Li J, Shi HM, Li Y.; Cytochrome P450 2C19 polymorphism is associated with poor clinical outcomes in coronary artery disease patients treated with clopidogrel. Mol Biol Rep. 2011 Mar;38(3):1697-702., Shuldiner AR, O'Connell JR, Bliden KP, Gandhi A, Ryan K, Horenstein RB, Damcott CM, Pakyz R, Tantry US, Gibson Q, Pollin TI, Post W, Parsa A, Mitchell BD, Faraday N, Herzog W, Gurbel PA.; Association of cytochrome P450 2C19 genotype with the antiplatelet effect and clinical efficacy of clopidogrel therapy. JAMA. 2009 Aug 26, 302(8):849-57.; Sibbing D, Stegherr J, Latz W, Koch W, Mehilli J, Dörrler K, Morath T, Schömig A, Kastrati A, von Beckerath N.; Cytochrome P450 2C19 loss-of-function polymorphism and stent thrombosis following percutaneous coronary intervention. Eur Heart J. 2009 Apr, 30(8):916-22.; Hulot JS, Bura A, Villard E, Azizi M, Remones V, Goyenvalle C, Aiach M, Lechat P, Gaussem P.; Cytochrome P450 2C19 loss-of-function polymorphism is a major determinant of clopidogrel responsiveness in healthy subjects. Blood. 2006 Oct 1, 108(7):2244-7.]. Mit Hilfe des erfindungsgemäßen Konzept kann auch in diesem Fall eine Geno- bzw. Phänotyp-spezifische Arzneimittelformulierung, vorzugsweise eine FDC, bestehend aus Clopidogrel und seinem aktiven Metaboliten berechnet werden, um die mangelnde Bildung an aktivem Metaboliten bei CYP2C19 PMs auszugleichen.

Zur Ermittlung des optimalen Referenzplasmaspiegels im Steady State können entweder ermittelten Daten verwendet werden, oder ein Pharmakokinetischen Model wie PK-Sim® und MoBi® eingesetzt werden, welches den Plasmaspiegel nach Eingabe einer Referenzdosis berechnen kann.

Darüber hinaus lässt sich das Prinzip einer Geno- bzw. Phänotyp-spezifischen Arzneimittelformulierung, vorzugsweise einer FDC, bestehend aus einer Muttersubstanz und einem oder mehreren Metaboliten, auf alle Muttersubstanzen, die über ein Enzym, Protein, Rezeptor oder Transporter, welches inhibiert/induziert werden kann, in einen oder mehrere aktiven Metaboliten umgewandelt, und/oder gebunden und/oder transportiert werden und/oder ihre pharmakodynamische Wirkung entfalten, übertragen.

Wie oben bereits ausführlich am Beispiel von Tamoxifen und CYP2D6-Inhibitor Paroxetin ausgeführt, kann die notwendige gleichzeitige Gabe eines Arzneistoffes A und eines Arzneistoffes B, wobei A über ein Enzym in einen aktiven Metaboliten umgewandelt werden muss, um seine vollständige Wirkung zu entfalten, und B dieses Enzym hemmt, ein Patient faktisch von einem EM Geno- bzw. Phänotyp in einen PM Geno- bzw. Phänotyp umwandeln. Durch die medizinisch indizierte gleichzeitige Verabreichung von Paroxetin wird der Patient faktisch in einen CYP2D6 PM umgewandelt, welcher entsprechend weniger Tamoxifen in Endoxifen umsetzen kann. Mit Hilfe des oben näher erläuterten Konzepts kann hier ebenfalls eine Geno- bzw. Phänotyp-spezifische Arzneimittelformulierung, vorzugsweise eine FDC, bestehend aus Tamoxifen und Endoxifen berechnet werden, welche den Mangel an Endoxifenbildung aus Tamoxifen durch die von Paroxetin verursachte Hemmung von CYP2D6 ausgleichen kann.

Analog wäre das Konzept anwendbar im Falle einer notwendigen und medizinisch indizierten gleichzeitigen Verabreichung von Clopidogrel und dem kompetitiven CYP2C19 Inhibitor Omeprazol. Eine daraus resultierende verminderte Umwandlung von Clopidogrel in seinen aktiven Metaboliten kann ebenfalls mit Hilfe der oben näher erläuterten Konzept und Methode durch die Berechnung einer Geno- bzw. Phänotyp-spezifische Arzneimittelformulierung, vorzugsweise einer FDC, bestehend aus Clopidogrel und seinem aktiven Metaboliten ausgeglichen werden.

Das oben erläuterte Konzept ist außerdem in der Lage eine Kombination aus einem genetischen Polymorphismus und einer Enzymhemmung und/oder Enzyminduktion, die additiv das gleiche oder unterschiedliche Enzyme oder Proteine oder Rezeptoren oder Transporter in ihrer Aktivität mindern/steigern, zu kompensieren. Exemplarisch erläutert wird dies am Beispiel eines Patienten mit einem CYP2D6 PM Geno- bzw. Phänotyp, der eine Tamoxifentherapie erhält und zusätzlich die Gabe von Paroxetin benötigt. Die Auswirkung auf die Bildung von Endoxifen aus Tamoxifen über CYP2D6 kann mit dem oben näher erläuterten Prinzip berücksichtigt werden und eine optimale Geno- bzw. Phänotyp-spezifische Arzneimittelformulierung, vorzugsweise eine FDC, bestehend aus Tamoxifen und Endoxifen berechnet werden. Analog lässt sich dieses auch am Beispiel eines Patienten mit einem CYP2C19 PM Geno- bzw. Phänotyp unter einer Clopidogrel-Therapie, der nun die Gabe von Omeprazol benötigt, nachvollziehen.

### Abbildungen:

Die Abbildungen erläutern das erfinderische Konzept für eine Tamoxifen-Therapie und zeigen die Ergebnisse der Tamoxifen/Endoxifen-FDC-Dosisfindung mit PK-Sim® nach dem erfindungsgemäßen Verfahren als Beispiel, ohne das Konzept auf dieses Beispiel zu begrenzen.

**Abbildung. 1** zeigt einen Ausschnitt aus dem komplexen Biotransformationsschema von Tamoxifen im Menschen. Tamoxifen wird etwa zu 90% zu N-Desmethyltamoxifen und zu ca 7% zu 4-Hydroxytamoxifen metabolisiert. Die Bildung von Endoxifen erfolgt aus N-Desmethyltamoxifen exklusiv über das polymorphe Cytochrom P450 (CYP) 2D6. Die Bildung von 4-Hydroxytamoxifen aus Tamoxifen erfolgt etwa zu 50% über das polymorphe CYP2D6. CYP2D6 ist damit in die essentiellen Bildungsschritte von Endoxifen zu einem hohen Anteil involviert [Coller, J. K., N. Krebsfaenger, et al. (2002). "The influence of CYP2B6, CYP2C9 and CYP2D6 genotypes on the formation of the potent antioestrogen Z-4-hydroxy-tamoxifen in human liver." Br J Clin Pharmacol 54(2): 157-167.; Desta, Z., B. A. Ward, et al. (2004). "Comprehensive evaluation of tamoxifen sequential biotransformation by the human cytochrome P450 system in vitro: prominent roles for CYP3A and CYP2D6." J Pharmacol Exp Ther 310(3): 1062-1075.; Kaku, T., K. Ogura, et al. (2004). "Quaternary ammonium-linked glucuronidation of tamoxifen by human liver microsomes and UDP-glucuronosyltransferase 1A4." Biochem Pharmacol 67(11): 2093-2102.; Murdter, T. E., W. Schroth, et al. (2011). "Activity levels of tamoxifen metabolites at the estrogen receptor and the impact of genetic polymorphisms of phase I and II enzymes on their concentration levels in plasma." Clin Pharmacol Ther 89(5): 708-717.; Nishiyama, T., K. Ogura, et al. (2002). "Reverse geometrical selectivity in glucuronidation and sulfation of cis- and trans-4-hydroxytamoxifens by human liver UDP-glucuronosyltransferases and sulfotransferases." Biochem Pharmacol 63(10): 1817-1830.; Sun, D., G. Chen, et al. (2006). "Characterization of tamoxifen and 4-hydroxytamoxifen glucuronidation by human UGT1A4 variants." Breast Cancer Res 8(4): R50.; Sun, D., A. K. Sharma, et al. (2007). "Glucuronidation of active tamoxifen metabolites by the human UDP glucuronosyltransferases." Drug Metab Dispos 35(11): 2006-2014.]

**Abbildung 2** zeigt Cytochrom P450 (CYP) 2D6 Geno- bzw. Phänotyp-abhängige Steady-State-Konzentrationen von Endoxifen im Rahmen einer Tamoxifentherapie bei Patientinnen vom CYP2D6 extensive metabolizer (EM), intermediate metabolizer (IM) bzw. poor metabolizer (PM) Phänotyp. Ein Gen-Dosis-Effekt der Endoxifenkonzentration ist erkennbar: Patientinnen mit zwei funktionalen CYP2D6 Allelen (EMs) zeigen eine deutlich höhere Endoxifenexposition als Patientinnen mit nur einem CYP2D6 funktionalen Allel (IMs) bzw. keinem funktionalem CYP2D6 Allel (PM). [Abbildungen aus (von links oben nach rechts unten): [Kiyotani, K., T. Mushiroda, et al. (2010). "Significant effect of polymorphisms in CYP2D6 and ABCC2 on clinical outcomes of adjuvant tamoxifen therapy for breast cancer patients." J Clin Oncol 28(8): 1287-1293.; Murdter, T. E., W. Schroth, et al. (2011). "Activity levels of tamoxifen metabolites at the estrogen receptor and the impact of genetic polymorphisms of phase I and II enzymes on their concentration levels in plasma." Clin Pharmacol Ther 89(5): 708-717.; Lim, J. S., X. A. Chen, et al. (2011). "Impact of CYP2D6, CYP3A5, CYP2C9 and CYP2C19 polymorphisms on tamoxifen pharmacokinetics in Asian breast cancer patients." Br J Clin Pharmacol 71(5): 737-750.; Lim, H. S., H. Ju Lee, et al. (2007). "Clinical implications of CYP2D6 genotypes predictive of tamoxifen pharmacokinetics in metastatic breast cancer." J Clin Oncol 25(25): 3837-3845.; Borges, S., Z. Desta, et al. (2006). "Quantitative effect of CYP2D6 genotype and inhibitors on tamoxifen metabolism: implication for optimization of breast cancer treatment." Clin Pharmacol Ther 80(1): 61-74.; Jin, Y., Z. Desta, et al. (2005). "CYP2D6 Genotype, Antidepressant Use, and Tamoxifen Metabolism During Adjuvant Breast Cancer Treatment." Journal ofthe National Cancer Institute 97(1): 30-39.]

**Abbildung** 3 zeigt Rezidiv-freie Überlebenskurven (nach Kaplan-Meier) für Brustkrebspatientinnen unter einer Tamoxifen-Therapie in Abhängigkeit vom Cytochrom P450 (CYP) 2D6 extensive metabolizer (EM, intermediate (IM), oder poor metabolizer (PM) Geno- bzw. Phänotyp. [Abbildungen aus (Reihe 1 bis 3): Schroth, W., M. P. Goetz, et al. (2009). "Association between CYP2D6 polymorphisms and outcomes among women with early stage breast cancer treated with tamoxifen." JAMA 302(13): 1429-1436.; Goetz, M. P., S. K. Knox, et al. (2007). "The impact of cytochrome P450 2D6 metabolism in women receiving adjuvant tamoxifen." Breast Cancer Res Treat 101(1): 113-121.; Goetz, M. P., J. M. Rae, et al. (2005). "Pharmacogenetics of tamoxifen biotransformation is associated with clinical outcomes of efficacy and hot flashes." J Clin Oncol 23(36): 9312-9318.]

**Abbildung 4** zeigt eine schematische Darstellung der Kompartimente des gekoppelten Physiologie-basierten Pharmakokinetik (PBPK)-Modells wie in PK-Sim® verwendet für die Simulation der Cytochrom P450 (CYP) 2D6 geno- bzw. phänotyp-spezifischen Bildung von N-Desmethyltamoxifen, 4-Hydroxytamoxifen und Endoxifen nach der Gabe der Muttersubstanz Tamoxifen bzw. für die Simulation der gleichzeitigen Gabe von Tamoxifen und Endoxifen in Abhängigkeit vom CYP2D6 Geno- bzw. Phänotyp und die daraus resultierenden Serumkonzentrationen. Im intrazellulären Kompartiment der Leber entstehen aus Tamoxifen N-Desmethyltamoxifen und 4-Hydroxytamoxifen, sodass das Tamoxifen-PBPK-Modell als Entstehungsfunktion der beiden primären Metabliten fungiert. Analog entsteht in den intrazellulären Kompartimenten der PBPK-Modelle von N-Desmethyltamoxifen und 4-Hydroxytamoxifen der sekundäre Metabolit Endoxifen.

Abbildung 5A zeigt gekoppelte PBPK-Modelle für Tamoxifen (TAM), N-Desmethyltamoxifen (NDM), 4-Hydroxytamoxifen (4OH), Endoxifen (END) in CYP2D6 extensive metabolizer, intermediate metabolizer und poor metabolizer (EM/IM/PM) Geno- bzw. Phänotyp Populationen. Steady-state Plasmakonzentrationen von Tamoxifen, N-Desmethyltamoxifen, 4-Hydroxytamoxifen und Endoxifen nach 1-mal-täglicher Gabe von 20 mg Tamoxifen über 1 Jahr in Beispielpopulationen von europäischen Frauen vom Cyotchrom P450 (CYP) 2D6 extensive metabolizer (EM), intermediate metabolizer (IM) und poor metabolizer (PM) Geno- bzw. Phänotyp. Box-whisker-Plots zeigen die Perzentilen 5, 25, 50, 75, und 95 der jeweiligen Populationen. Symbole repräsentieren experimentelle Daten für die Modellvalidierung [von links nach rechts: Gjerde, J. Geisler, et al. (2010). "Associations between tamoxifen, estrogens, and FSH serum levels during steady state tamoxifen treatment of postmenopausal women with breast cancer." BMC Cancer 10: 313.; Gjerde, J., M. Hauglid, et al. (2008). "Effects of CYP2D6 and SULT1A1 genotypes including SULT1A1 gene copy number on tamoxifen metabolism." Ann Oncol 19(1): 56-61.; Madlensky, L., L. Natarajan, et al. (2011). "Tamoxifen metabolite concentrations, CYP2D6 genotype, and breast cancer outcomes." Clin Pharmacol Ther 89(5): 718-725.; Murdter, T. E., W. Schroth, et al. (2011). "Activity levels of tamoxifen metabolites at the estrogen receptor and the impact of genetic polymorphisms of phase I and II enzymes on their concentration levels in plasma." Clin Pharmacol Ther 89(5): 708-717.; Irvin, W. J., Jr., C. M. Walko, et al. (2011). "Genotype-Guided Tamoxifen Dosing Increases Active Metabolite Exposure in Women With Reduced CYP2D6 Metabolism: A Multicenter Study." J Clin Oncol 29(24): 3232-3239.]. **Abbildung. 5B** stellt eine alternative Darstellung dar.

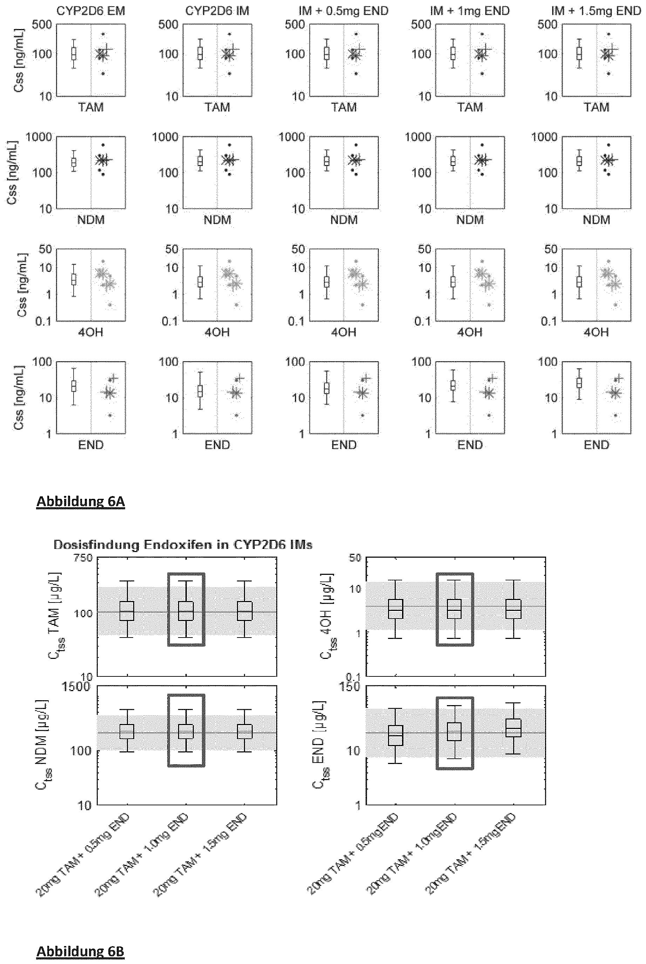

**Abbildung 6A** zeigt das Ergebniss der Endoxifen-Dosisfindung mit PK-Sim® nach dem erfindungsgemäßen Verfahren für die gleichzeitige Gabe mit Tamoxifen in CYP2D6 IM Patienten. **Abbildung. 6A** zeigt Steady-State Plasmakonzentrationen von Tamoxifen (TAM), N-Desmethyltamoxifen (NDM), 4-Hydroxytamoxifen (4OH) und Endoxifen (END) nach 1-mal-täglicher Gabe von 20 mg Tamoxifen täglich über 1 Jahr in Beispielpopulationen von europäischen Patientinnen mit dem Cytochrom P450 (CYP) 2D6 extensive metabolizer (EM) bzw. intermediate metabolizer (IM) Geno- bzw. Phänotyp im Vergleich zu experimentellen Daten von Patientinnen des CYP2D6 EM Geno- bzw. Phänotyps. Steady-State Plasmakonzentrationen von Tamoxifen, N-Desmethyltamoxifen, 4-Hydroxytamoxifen und Endoxifen in Beispielpopulationen von europäischen Patientinnen des CYP2D6 IM Geno bzw. Phänotyps nach gleichzeitiger 1-mal-täglicher Gabe von 20 mg Tamoxifen plus 0.5 mg bzw. 1 mg bzw. 1.5 mg Endoxifen zusätzlich über 1 Jahr. CYP2D6 IM Patientinnen die dabei 20 mg Tamoxifen plus 1 mg Endoxifen erhielten zeigten dabei äquivalente Endoxifenkonzentrationen zu CYP2D6 EM Patientinnen, die 20 mg Tamoxifen 1-mal täglich über 1 Jahr erhielten. [von links nach rechts: Gjerde, J. Geisler, et al. (2010). "Associations between tamoxifen, estrogens, and FSH serum levels during steady state tamoxifen treatment of postmenopausal women with breast cancer." BMC Cancer 10: 313.; Gjerde, J., M. Hauglid, et al. (2008). "Effects of CYP2D6 and SULT1A1 genotypes including SULT1A1 gene copy number on tamoxifen metabolism." Ann Oncol 19(1): 56-61.; Madlensky, L., L. Natarajan, et al. (2011). "Tamoxifen metabolite concentrations, CYP2D6 genotype, and breast cancer outcomes." Clin Pharmacol Ther 89(5): 718-725.; Murdter, T. E., W. Schroth, et al. (2011). "Activity levels of tamoxifen metabolites at the estrogen receptor and the impact of genetic polymorphisms of phase I and II enzymes on their concentration levels in plasma." Clin Pharmacol Ther 89(5): 708-717.; Irvin, W. J., Jr., C. M. Walko, et al. (2011). "Genotype-Guided Tamoxifen Dosing Increases Active Metabolite Exposure in Women With Reduced CYP2D6 Metabolism: A Multicenter Study." J Clin Oncol 29(24): 3232-3239] **Abbildung 6B** stellt eine alternative Darstellung dar. Als Vergleich dienen die ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). CYP2D6 IM Patientinnen die dabei 20 mg Tamoxifen plus 1 mg Endoxifen erhielten zeigten dabei äquivalente Endoxifenkonzentrationen zu CYP2D6 EM Patientinnen, die 20 mg Tamoxifen 1-mal täglich über 1 Jahr erhielten.

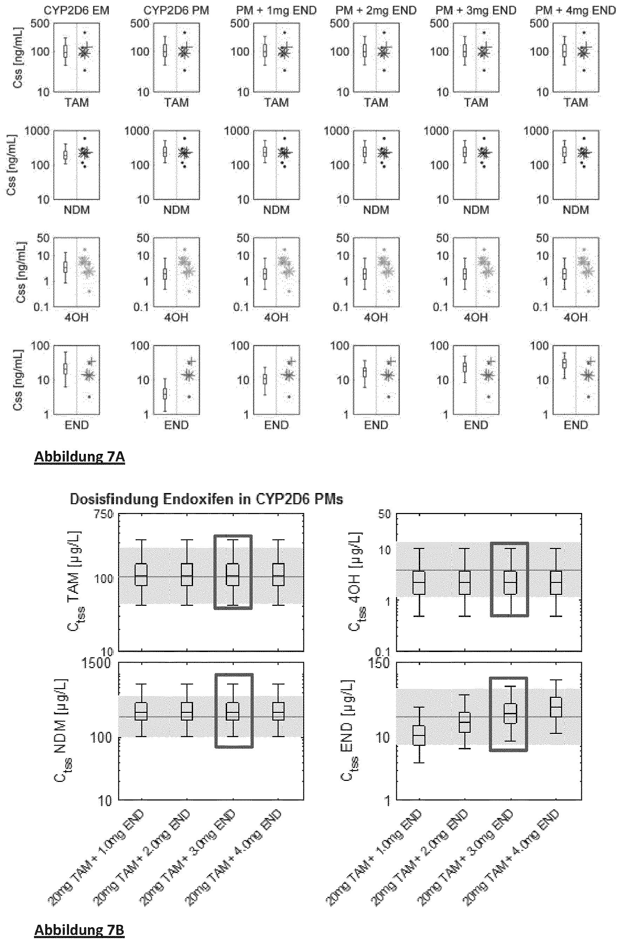

**Abbildung 7A** zeigt das Ergebnis der Endoxifen-Dosisfindung mit PK-Sim® nach dem erfindungsgemäßen Verfahren für die gleichzeitige 1-mal-tägliche Gabe mit Tamoxifen in CYP2D6 PM Patienten. **Abbildung 7A** zeigt Steady-State Plasmakonzentrationen von Tamoxifen (TAM), N-Desmethyltamoxifen (NDM), 4-Hydroxytamoxifen (4OH) und Endoxifen (END) nach Gabe von 20 mg Tamoxifen 1-mal täglich über 1 Jahr in Beispielpopulationen von europäischen Patientinnen mit dem Cytochrom P450 (CYP) 2D6 extensive metabolizer (EM) bzw. poor metabolizer (PM) Geno- bzw. Phänotyp im Vergleich zu experimentellen Daten von Patientinnen des CYP2D6 EM Geno- bzw. Phänotyps. Steady-State Plasmakonzentrationen von Tamoxifen, N-Desmethyltamoxifen, 4-Hydroxytamoxifen und Endoxifen in Beispielpopulationen von europäischen Patientinnen des CYP2D6 PM Geno bzw. Phänotyps nach gleichzeitiger Gabe von 20 mg Tamoxifen plus 1 mg bzw. 2 mg bzw. 3 mg bzw. 4 mg Endoxifen zusätzlich über 1 Jahr. CYP2D6 PM Patientinnen die dabei 20 mg Tamoxifen plus 3 mg Endoxifen erhielten zeigten dabei äquivalente Endoxifenkonzentrationen zu CYP2D6 EM Patientinnen, die 20 mg Tamoxifen 1-mal täglich über 1 Jahr erhielten. [von links nach rechts: Gjerde, J. Geisler, et al. (2010). "Associations between tamoxifen, estrogens, and FSH serum levels during steady state tamoxifen treatment of postmenopausal women with breast cancer." BMC Cancer 10: 313.; Gjerde, J., M. Hauglid, et al. (2008). "Effects of CYP2D6 and SULT1A1 genotypes including SULT1A1 gene copy number on tamoxifen metabolism." Ann Oncol 19(1): 56-61.; Madlensky, L., L. Natarajan, et al. (2011). "Tamoxifen metabolite concentrations, CYP2D6 genotype, and breast cancer outcomes." Clin Pharmacol Ther 89(5): 718-725.; Murdter, T. E., W. Schroth, et al. (2011). "Activity levels of tamoxifen metabolites at the estrogen receptor and the impact of genetic polymorphisms of phase I and II enzymes on their concentration levels in plasma." Clin Pharmacol Ther 89(5): 708-717.; Irvin, W. J., Jr., C. M. Walko, et al. (2011). "Genotype-Guided Tamoxifen Dosing Increases Active Metabolite Exposure in Women With Reduced CYP2D6 Metabolism: A Multicenter Study." J Clin Oncol 29(24): 3232-3239.] **Abbildung 7B** stellt eine alternative Darstellung dar. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). CYP2D6 PM Patientinnen die dabei 20 mg Tamoxifen plus 3 mg Endoxifen erhielten zeigten dabei äquivalente Endoxifenkonzentrationen zu CYP2D6 EM Patientinnen, die 20 mg Tamoxifen 1-mal täglich über 1 Jahr erhielten.

**Abbildung 8** zeigt Genotyp- bzw. Phänotyp-basierte Dosierung von Tamoxifen und Endoxifen als lose Kombination (A) oder als FDC (B)

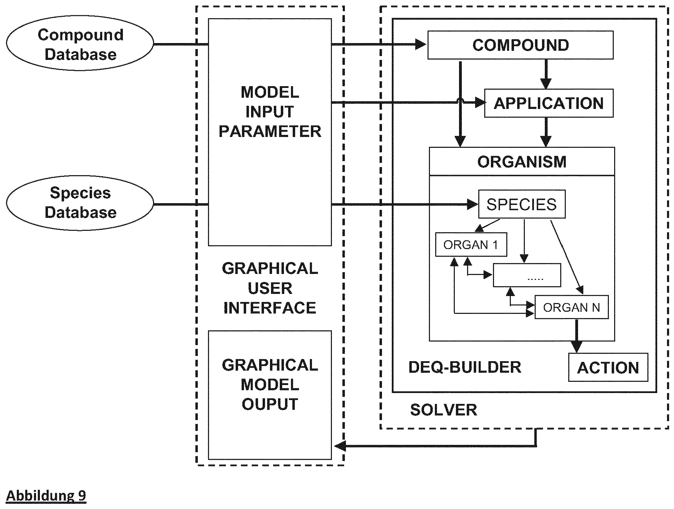

**Abbildungen 9 und 10** zeigt eine schematische Darstellung des Modulaufbaus von PK-Sim®.

**Abbildungen 11 bis 14** zeigen den Einfluss einer initialen Brustkrebs-Therapie mit der fixen Kombination aus 20 mg Tamoxifen und 3 mg Endoxifen auf das Erreichen der Endoxifen Steady-State Konzentrationen systematisch untersucht mittels des PBPK-Modells für CYP2D6 EMs und IMs.

**Abbildung 11** zeigt das Ergebnis der Loading-Dose-Studie mit PK-Sim® nach dem erfindungsgemäßen Verfahren für die gleichzeitige Gabe von Tamoxifen und Endoxifen in CYP2D6 EM Patienten. **Abbildung 11** zeigt die Talplasmakonzentrationen von Tamoxifen (TAM), N-Desmethyltamoxifen (NDM), 4-Hydroxytamoxifen (4OH) und Endoxifen (END) nach gleichzeitiger 1 mal-täglicher Gabe von 20 mg Tamoxifen und 3 mg Endoxifen in europäischen Patientinnen mit dem Cytochrom P450 (CYP) 2D6 extensive metabolizer (EM) Geno- bzw. Phänotyp. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde dabei der Vortag zu dem Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie überschreitet, in diesem Fall Tag 9.

**Abbildung 12** zeigt das Ergebnis der Loading-Dose-Kontroll-Studie mit PK-Sim® nach dem erfindungsgemäßen Verfahren für die gleichzeitige Gabe von Tamoxifen und Endoxifen in CYP2D6 EM Patienten. **Abbildung 12** zeigt die Talplasmakonzentrationen von Tamoxifen (TAM), N-Desmethyltamoxifen (NDM), 4-Hydroxytamoxifen (4OH) und Endoxifen (END) nach gleichzeitiger 1 mal-täglicher Gabe von 20 mg Tamoxifen in europäischen Patientinnen mit dem Cytochrom P450 (CYP) 2D6 extensive metabolizer (EM) Geno- bzw. Phänotyp. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde der Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie erreicht, in diesem Fall Tag 120.

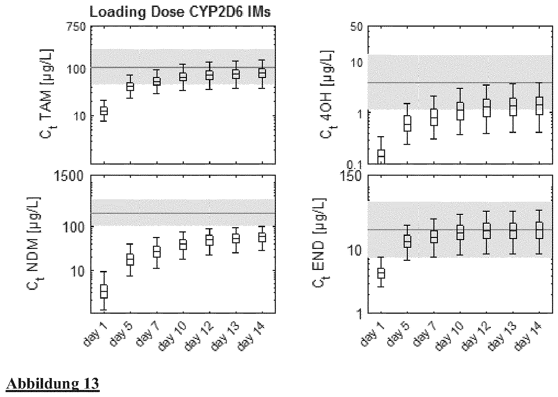

**Abbildung 13** zeigt das Ergebnis der Loading-Dose-Studie mit PK-Sim® nach dem erfindungsgemäßen Verfahren für die gleichzeitige Gabe von Tamoxifen und Endoxifen in CYP2D6 IM Patienten. **Abbildung 13** zeigt die Talplasmakonzentrationen von Tamoxifen (TAM), N-Desmethyltamoxifen (NDM), 4-Hydroxytamoxifen (4OH) und Endoxifen (END) nach gleichzeitiger 1 mal-täglicher Gabe von 20 mg Tamoxifen und 3 mg Endoxifen in europäischen Patientinnen mit dem Cytochrom P450 (CYP) 2D6 intermediate metabolizer (IM) Geno- bzw. Phänotyp. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde dabei der Vortag zu dem Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie überschreitet, in diesem Fall Tag 13.

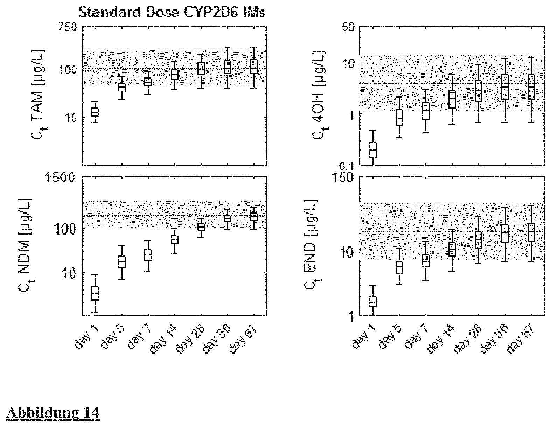

**Abbildung 14** zeigt das Ergebnis der Loading-Dose-Kontroll-Studie mit PK-Sim® nach dem erfindungsgemäßen Verfahren für die gleichzeitige Gabe von Tamoxifen und Endoxifen in CYP2D6 IM Patienten. Abbildung 14 zeigt die Talplasmakonzentrationen von Tamoxifen (TAM), N-Desmethyltamoxifen (NDM), 4-Hydroxytamoxifen (4OH) und Endoxifen (END) nach gleichzeitiger 1 mal-täglicher Gabe von 20 mg Tamoxifen und 1 mg Endoxifen in europäischen Patientinnen mit dem Cytochrom P450 (CYP) 2D6 intermediate metabolizer (IM) Geno- bzw. Phänotyp. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde der Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie erreicht, in diesem Fall Tag 67.

Zusammenfassend zeigt der direkte Vergleich zwischen der Gabe von 20 mg Tamoxifen in CYP2D6 EMs bzw. 20 mg Tamoxifen und 1 mg Endoxifen gemäß der Erfindung in IMs und der erfindungsgemäßen Gabe von 20 mg Tamoxifen und 3 mg Endoxifen in CYP2D6 EMs bzw. IMs deutlich, dass die Endoxifen Steady-State Konzentration mit der Gabe der FDC (bestehend aus 20 mg Tamoxifen und 3 mg Endoxifen) wesentlich schneller, im Schnitt etwa 111 Tage bzw. 54 Tage schneller erreicht wird als mit der Standarddosis (bestehend aus 20 mg Tamoxifen für EMs und 20 mg Tamoxifen und 1 mg Endoxifen gemäß der Erfindung).

**Abbildungen 15 bis 18** zeigen Simulationen bei der Untersuchtung von Compliance-Mängel. Die folgende Szenarien wurden simuliert:
**Abbildung 15** zeigt das Ergebnis der Compliance-Dose-Studie mit PK-Sim® nach dem erfindungsgemäßen Verfahren für die gleichzeitige Gabe von Tamoxifen und Endoxifen in CYP2D6 EM Patienten. **Abbildung 15** zeigt die Talplasmakonzentrationen von Tamoxifen (TAM), N-Desmethyltamoxifen (NDM), 4-Hydroxytamoxifen (4OH) und Endoxifen (END) nach Gabe von 20 mg Tamoxifen einmal täglich für 6 Monate und Einnahmepausen von 2, 4, 8, und 12 Wochen Dauer in europäischen Patientinnen mit dem Cytochrom P450 (CYP) 2D6 extensive metabolizer (EM) Geno- bzw. Phänotyp. Anschließend erfolgte die gleichzeitige 1 mal-tägliche Gabe von 20 mg Tamoxifen und 3 mg Endoxifen. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde dabei der Vortag zu dem Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie überschreitet, in diesem Fall Tag 2 nach Beginn der Einnahme der FDC bei 2 Wochen Einnahmepause, Tag 3 nach Beginn der Einnahme der FDC bei 4 Wochen Einnahmepause, Tag 7 nach Beginn der Einnahme der FDC bei 8 Wochen Einnahmepause und Tag 9 nach Beginn der Einnahme der FDC bei 12 Wochen Einnahmepause.

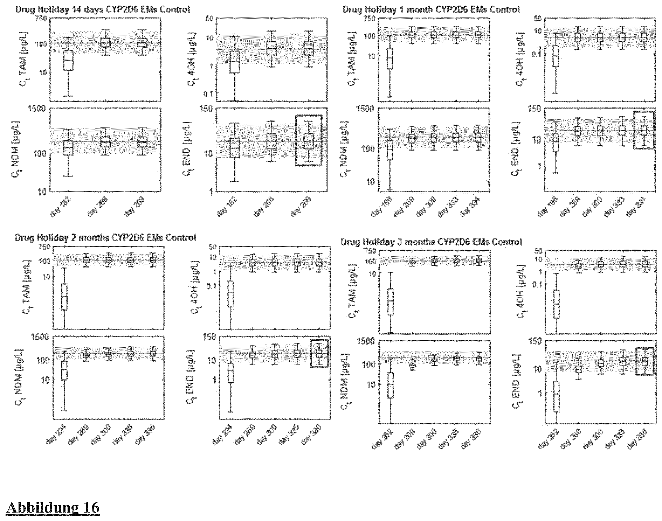

Abbildung 16 zeigt das Ergebnis der Compliance-Dose-Kontroll-Studie mit PK-Sim® nach dem erfindungsgemäßen Verfahren für die gleichzeitige Gabe von Tamoxifen und Endoxifen in CYP2D6 EM Patienten. Abbildung 16 zeigt die Talplasmakonzentrationen von Tamoxifen (TAM), N-Desmethyltamoxifen (NDM), 4-Hydroxytamoxifen (4OH) und Endoxifen (END) nach Gabe von 20 mg Tamoxifen einmal täglich für 6 Monate und Einnahmepausen von 2, 4, 8, und 12 Wochen Dauer in europäischen Patientinnen mit dem Cytochrom P450 (CYP) 2D6 extensive metabolizer (EM) Geno- bzw. Phänotyp. Anschließend erfolgte die 1 mal-tägliche Gabe von 20 mg Tamoxifen. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde dabei der Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie erreicht, in diesem Fall Tag 269 nach Beginn der Einnahme der FDC bei 2 Wochen Einnahmepause, Tag 334 nach Beginn der Einnahme der FDC bei 4 Wochen Einnahmepause, Tag > 336 nach Beginn der Einnahme der FDC bei 8 Wochen Einnahmepause und Tag > 336 nach Beginn der Einnahme der FDC bei 12 Wochen Einnahmepause.

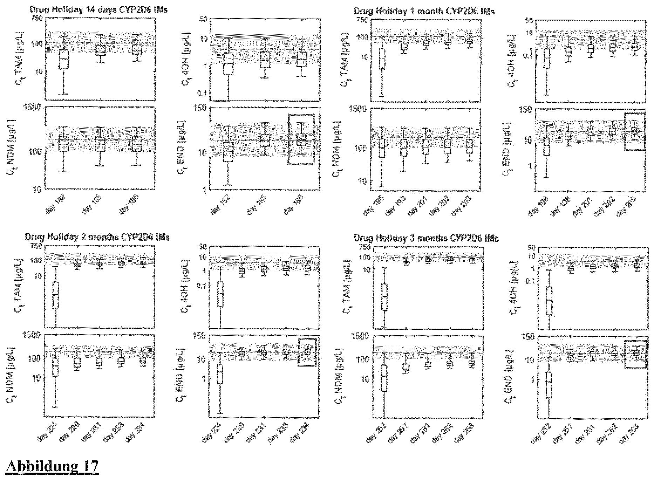

**Abbildung 17** zeigt das Ergebnis der Compliance-Dose-Studie mit PK-Sim® nach dem erfindungsgemäßen Verfahren für die gleichzeitige Gabe von Tamoxifen und Endoxifen in CYP2D6 IM Patienten. **Abbildung 17** zeigt die Talplasmakonzentrationen von Tamoxifen (TAM), N-Desmethyltamoxifen (NDM), 4-Hydroxytamoxifen (4OH) und Endoxifen (END) nach gleichzeitiger Gabe von 20 mg Tamoxifen und 1 mg Endoxifen einmal täglich für 6 Monate und Einnahmepausen von 2, 4, 8, und 12 Wochen Dauer in europäischen Patientinnen mit dem Cytochrom P450 (CYP) 2D6 intermediate metabolizer (IM) Geno- bzw. Phänotyp. Anschließend erfolgte die gleichzeitige 1 mal-täglicher Gabe von 20 mg Tamoxifen und 3 mg Endoxifen. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde dabei der Vortag zu dem Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie überschreitet, in diesem Fall Tag 4 nach Beginn der Einnahme der FDC bei 2 Wochen Einnahmepause, Tag 7 nach Beginn der Einnahme der FDC bei 4 Wochen Einnahmepause, Tag 10 nach Beginn der Einnahme der FDC bei 8 Wochen Einnahmepause und Tag 11 nach Beginn der Einnahme der FDC bei 12 Wochen Einnahmepause.

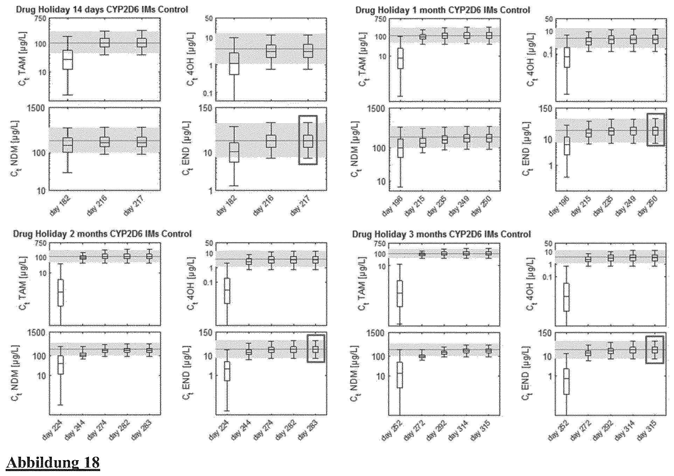

**Abbildung 18** zeigt das Ergebnis der Compliance-Dose-Kontroll-Studie mit PK-Sim® nach dem erfindungsgemäßen Verfahren für die gleichzeitige Gabe von Tamoxifen und Endoxifen in CYP2D6 IM Patienten. **Abbildung 18** zeigt die Talplasmakonzentrationen von Tamoxifen (TAM), N-Desmethyltamoxifen (NDM), 4-Hydroxytamoxifen (4OH) und Endoxifen (END) nach gleichzeitiger Gabe von 20 mg Tamoxifen und 1 mg Endoxifen einmal täglich für 6 Monate und Einnahmepausen von 2, 4, 8, und 12 Wochen Dauer in europäischen Patientinnen mit dem Cytochrom P450 (CYP) 2D6 intermediate metabolizer (IM) Geno- bzw. Phänotyp. Anschließend erfolgte die 1 mal-täglicher gleichzeitige Gabe von 20 mg Tamoxifen und 1 mg Endoxifen. Als Vergleich dienen die zuvor ermittelten Steady-State Talplasmakonzentrationen von Tamoxifen (TAM), N-Desmethyltamoxifen (NDM), 4-Hydroxytamoxifen (4OH) und Endoxifen (END) in europäischen Patientinnen des CYP2D6 EM Geno bzw. Phänotyps nach 1-mal-täglicher Gabe von 20 mg Tamoxifen über 1 Jahr, dargestellt als graues Band (Perzentile 5-95) mit Median (dunkelgraue Linie). Als Zeitpunkt wurde dabei der Tag angenommen, an dem der mediane Talspiegelwert der Endoxifenkonzentration erstmals die mediane Talspiegel-Endoxifenkonzentration in der Beispielpopulation bestehend aus europäischen Patientinnen vom CYP2D6 EM Geno- bzw. Phänotyp unter Standardtherapie erreicht, in diesem Fall Tag 217 nach Beginn der Einnahme der FDC bei 2 Wochen Einnahmepause, Tag 250 nach Beginn der Einnahme der FDC bei 4 Wochen Einnahmepause, Tag 283 nach Beginn der Einnahme der FDC bei 8 Wochen Einnahmepause und Tag 315 nach Beginn der Einnahme der FDC bei 12 Wochen Einnahmepause.

Zusammenfassend zeigen die Simulationsergebnisse von **Abbildung** 15 bis 18, dass die fixe kombinierte Gabe von 20 mg Tamoxifen und 3 mg Endoxifen vorteilhaft ist, um im Falle von Compliance-Mängeln das Erreichen der wirksamen Steady-State Konzentrationen von Endoxifen zu beschleunigen.

## Patentansprüche

1. Fixed·Dose·Combination Arzneitmittelformulierung enthaltend eine Muttersubstanz Tamoxifen, deren Wirkung von der Menge bzw. der Aktivität von exprimierten Proteinvarianten, Enzymvarianten, Rezeptorvarianten oder Transportervarianten abhängig ist, und mindestens Endoxifen, einen der Metaboliten der Muttersubstanz Tamoxifen, umfassend 15 - 25 mg Tamoxifen und 0,25 -5,0 mg Endoxifen.

2. Formulierung nach Anspruch 1 für CYP2D6 IM Patienten umfassend 15 - 25 mg Tamoxifen und 0,25 - 2,00 mg Endoxifen.

3. Formulierung nach Anspruch 1 für CYP2D6 PM Patienten umfassend 15 - 25 mg Tamoxifen und 1,0 - 5,0 mg Endoxifen.

## Claims

1. Fixed-dose combination pharmaceutical formulation containing a tamoxifen parent substance, the action of which is dependent on the quantity or the activity of expressed protein variants, enzyme variants, receptor variants or transporter variants, and at least endoxifen, one of the metabolites of the tamoxifen parent substance, comprising 15-25 mg of tamoxifen and 0.25-5.0 mg of endoxifen.

2. Formulation according to Claim 1 for CYP2D6 IM patients comprising 15-25 mg of tamoxifen and 0.25-2.00 mg of endoxifen.

3. Formulation according to Claim 1 for CYP2D6 PM patients comprising 15-25 mg of tamoxifen and 1.0-5.0 mg of endoxifen.

## Revendications

1. Formulation de médicament combinée à dose fixe contenant une substance mère de tamoxifène, dont l'effet dépend de la quantité ou de l'activité des variants de protéine, des variants d'enzyme, des variants de récepteur ou des variants de transporteur exprimés et au moins de l'endoxifène, un des métabolites de la substance mère de tamoxifène, contenant 15-25 mg de tamoxifène et 0,25-5,0 mg d'endoxifène.

2. Formulation selon la revendication 1 pour des patients porteurs de CYP2D6 IM contenant 15-25 mg de tamoxifène et 0,25-2,00 mg d'endoxifène.

3. Formulation selon la revendication 1 pour des patients porteurs de CYP2D6 IM contenant 15-25 mg de tamoxifène et 1,0-5,0 mg d'endoxifène.
